(19) Europäisches Patentamt / European Patent Office / Office européen des brevets

(11) **EP 4 321 176 A1**

(12) **EUROPEAN PATENT APPLICATION**
published in accordance with Art. 153(4) EPC

(43) Date of publication:
**14.02.2024  Bulletin 2024/07**

(21) Application number: **22775732.5**

(22) Date of filing: **24.03.2022**

(51) International Patent Classification (IPC):
**A61K 45/00** *(2006.01)*      **A61P 9/12** *(2006.01)*
**A61P 11/00** *(2006.01)*      **G01N 33/53** *(2006.01)*

(52) Cooperative Patent Classification (CPC):
**A61K 45/00; A61P 9/12; A61P 11/00; G01N 33/53**

(86) International application number:
**PCT/JP2022/013767**

(87) International publication number:
**WO 2022/202950 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **25.03.2021   JP 2021051574**

(71) Applicants:
• **CHUGAI SEIYAKU KABUSHIKI KAISHA**
  **Tokyo 115-8543 (JP)**

• **International University Of Health And Welfare Ohtawara-shi, Tochigi 324-8501 (JP)**

(72) Inventor: **TAMURA, Yuichi**
**Tokyo 108-8329 (JP)**

(74) Representative: **Vossius & Partner Patentanwälte Rechtsanwälte mbB Siebertstrasse 3 81675 München (DE)**

Remarks:
The complete document including Reference Table(s) and the Sequence Listing(s) can be downloaded from the EPO website

(54) **PULMONARY ARTERIAL HYPERTENSION PATIENT SELECTION METHOD AND BIOMARKER**

(57)      The present invention relates to a biomarker for predicting a responsiveness to a treatment targeting an IL-6 signaling pathway in a patient with pulmonary arterial hypertension and a method for selecting a patient.

EP 4 321 176 A1

## Description

[Technical Field]

The present invention relates to a biomarker for predicting a responsiveness to a treatment targeting an IL-6 signaling pathway in a patient with pulmonary arterial hypertension and a method for selecting a patient.

[Background Art]

Pulmonary arterial hypertension is an intractable disease designated by the Ministry of Health, Labour and Welfare, and has thousands of patients in Japan. Pulmonary arterial hypertension is pulmonary hypertension that exhibits an increase in pulmonary arterial pressure especially due to stenosis and occlusion caused by contraction, thickening, thrombosis, and the like in the pulmonary artery. Pulmonary arterial hypertension develops heart failure due to the progression of symptoms, leading to death. The survival time after diagnosis or onset is generally short, that is, the prognosis is poor.

Pulmonary hypertension is related to its cause and is clinically classified into 1) idiopathic pulmonary arterial hypertension, 2) hereditary pulmonary arterial hypertension, 3) drug/toxicant-induced pulmonary hypertension, and 4) pulmonary arterial hypertension associated with various diseases (connective tissue disease, HIV infection, portal hypertension, congenital heart disease, and schistosomiasis) (Guidelines for Treatment of Pulmonary Hypertension (revised version in 2017)). However, the detailed mechanism of the occurrence of stenosis or occlusion in the pulmonary artery is not yet known. The current treatment is performed in combination with symptomatic therapies such as pharmaceuticals that improve pulmonary artery stenosis and occlusion and dilate blood vessels, pharmaceuticals that regulate body fluids, oxygen inhalation, pulmonary transplantation, and the like, depending on its progression.

Recently, it has been reported that in pulmonary arterial hypertension, an increase in blood levels of inflammatory cytokines including interleukin-6 (IL-6) is correlated with the progression of pulmonary arterial hypertension and poor prognosis (Non Patent Literature 1), that the IL-6 receptor is highly expressed in pulmonary arterial smooth muscle cells, and significant improvement in pulmonary arterial hypertension is observed by inhibiting IL-6 action with anti-IL-6 receptor antibodies (Non Patent Literature 2), and the like. That is, it has been reported that IL-6 in blood is elevated and expression of IL-6 receptors in diseased organs is high in pulmonary arterial hypertension, and that treatments with IL-6 signaling pathway inhibitors are effective in pulmonary arterial hypertension.

The IL-6 in blood and/or IL-6 receptors in organs in patients are direct targets for treatments with an IL-6 signaling pathway inhibitor such as an anti-IL-6 antibody or an anti-IL-6 receptor antibody. That is, as described above, it is recognized that pulmonary arterial hypertension can be treated by inhibition of IL-6 action, and the content of IL-6 in patients with pulmonary arterial hypertension would be an indicator of responsiveness to a treatment with an IL-6 signaling pathway inhibitor.

IL-6, also referred to as B-cell stimulator 2 (BSF2), interferon $\beta$2 or the like, is a cytokine that binds to the IL-6 receptor on the cell membrane to form an IL-6/IL-6 receptor complex and then binds to gp130, thereby transmitting the biological activity signal of IL-6 into the cell (Non Patent Literature 3).

In recent years, in medical settings, efforts aimed at so-called personalized medicine, which provides optimal treatment suitable for individual patients, have been extensive in various disease fields. Also in patients with pulmonary arterial hypertension, it is desirable to appropriately select patients who are responsive to a treatment targeting the IL-6 signaling pathway, then apply the treatment.

[Citation List]

[Non Patent Literature]

[Non Patent Literature 1] Ectopic upregulation of membrane-bound IL6R drives vascular remodeling in pulmonary arterial hypertension, Yuichi Tamura et al., J. Clin. Invest., Vol. 128, No. 5, May 2018,1956-1970
[Non Patent Literature 2] Interleukin-6/interleukin-21 signaling axis is critical in the pathogenesis of pulmonary arterial hypertension, Takahiro Hashimoto-Kataoka et al., PNAS, Published online May 4, 2015, E2677-E2686
[Non Patent Literature 3] Interleukin-6 triggers the association of its receptor with a possible signal transducer, gp130, Tetsuya Taga et al., Cell, Vol. 58, Issue 3, August 11, 1989, 573-581

[Summary of Invention]

The present inventors have found a biomarker for predicting a content of IL-6, and a biomarker for predicting a responsiveness to a treatment with an IL-6 signaling pathway inhibitor and/or for selecting a subject who is likely to have a responsiveness to a treatment with an IL-6 signaling pathway inhibitor.

The present inventors have further found a method for selecting a novel biomarker, and a method for selecting a biomarker for predicting a responsiveness of a subject to a treatment with an IL-6 signaling pathway inhibitor and/or for selecting a subject who is likely to have a responsiveness to a treatment with an IL-6 signaling pathway inhibitor.

Based on these findings, the following inventions are provided.

[1-1] A method for selecting a novel biomarker, comprising:

(a) selecting a biomarker candidate substance based on a vector similarity acquired using a content of a reference biomarker and a content of the biomarker candidate substance in a sample collected from a subject group; and
(b) clustering the subject group based on the content of the biomarker candidate substance selected in (a).

[1-2] The selecting method according to [1-1], further comprising (c) selecting a biomarker candidate substance selected in (a) as a biomarker candidate substance when the biomarker candidate substance selected in (a) is capable of discriminating a subject with high reliability in comparison with a result of clustering in (b).

[1-3] The selecting method according to [1-2], comprising, in (c),

(d) comparing a result of discriminating the subject using each of a series of any threshold candidate values of the biomarker candidate substance selected in (a) with the result of clustering in (b), thereby acquiring a sensitivity and a specificity of the discrimination using each threshold candidate value of the biomarker candidate substance;
(e) acquiring a reliability based on the sensitivity and the specificity acquired in (d); and
(f) determining whether or not the biomarker candidate substance is capable of discriminating the subject with high reliability.

[1 -4] The selecting method according to any of [1-1] to [1-3], wherein the vector similarity is a cosine similarity.

[1-5] The selecting method according to any of [1-1] to [1-4], wherein the biomarker candidate substance is selected based on the vector similarity of about 0.6 or more.

[1-6] The selecting method according to any of [1-1] to [1-5], wherein the subject group is clustered by K-medoids method.

[1-7] The selecting method according to any of [1-1] to [1-6], wherein the subject group is clustered into three clusters.

[1-8] The selecting method according to any of [1-2] to [1-7], wherein the reliability is indicated by AUC of a ROC curve acquired based on the sensitivity and the specificity.

[1-9] The selecting method according to [1-8], wherein the biomarker candidate substance is determined to be capable of discriminating the subject with high reliability when the AUC is about 0.8 or more.

[1-10] The selecting method according to any of [1-1] to [1-9], which is a computer-implemented method.

[2-1] A device for selecting a novel biomarker, comprising:

(a) a selection unit that selects a biomarker candidate substance based on a vector similarity acquired using a content of a reference biomarker and a content of the biomarker candidate substance in a sample collected from a subject group; and
(b) a clustering unit that clusters the subject group based on the content of the biomarker candidate substance selected in (a).

[2-2] The selecting device according to [2-1], further comprising (c) a selection unit that selects a biomarker candidate substance selected in (a) as a biomarker candidate substance when the biomarker candidate substance selected in (a) is capable of discriminating a subject with high reliability in comparison with a result of clustering in (b).

[2-3] The selecting device according to [2-2], wherein the selection unit (c) comprises:

(d) an acquisition unit that compares a result of discriminating the subject using each of a series of any threshold candidate values of the biomarker candidate substance selected in (a) with the result of clustering in (b), thereby acquiring a sensitivity and a specificity of the discrimination using each threshold candidate value of the biomarker candidate substance;
(e) an acquisition unit that acquires a reliability based on the sensitivity and the specificity acquired in (d); and
(f) a determination unit that determines whether or not the biomarker candidate substance is capable of discriminating the subject with high reliability.

[2-4] The selecting device according to any of [2-1] to [2-3], wherein the vector similarity is a cosine similarity.

[2-5] The selecting device according to any of [2-1] to [2-4], wherein the biomarker candidate substance is selected based on the vector similarity of about 0.6 or more.

[2-6] The selecting device according to any of [2-1] to [2-5], wherein the subject group is clustered by K-medoids method.

[2-7] The selecting device according to any of [2-1] to [2-6], wherein the subject group is clustered into three clusters.

[2-8] The selecting device according to any of [2-2] to [2-7], wherein the reliability is indicated by AUC of a ROC curve acquired based on the sensitivity and the specificity.

[2-9] The selecting device according to [2-8], wherein the biomarker candidate substance is determined to be capable of discriminating the subject with high reliability when the AUC is about 0.8 or more.

[3-1] A method for selecting a biomarker for predicting a content of IL-6 in a subject, comprising:

(a) selecting a biomarker candidate substance based on a vector similarity acquired using a content of IL-6 and a content of the biomarker candidate substance in a sample collected from a subject group; and
(b) clustering the subject group based on the content of the biomarker candidate substance selected in (a).

[3-2] A method for selecting a biomarker for predicting a responsiveness of a subject to a treatment with an IL-6 signaling pathway inhibitor, comprising:

(a) selecting a biomarker candidate substance based on a vector similarity acquired using a content of IL-6 and a content of a biomarker candidate substance in a sample collected from a subject group; and
(b) clustering the subject group based on the content of the biomarker candidate substance selected in (a).

[3-3] A method for selecting a biomarker for selecting a subject who is likely to have a responsiveness to a treatment with an IL-6 signaling pathway inhibitor, comprising:

(a) selecting a biomarker candidate substance based on a vector similarity acquired using a content of IL-6 and a content of a biomarker candidate substance in a sample collected from a subject group; and
(b) clustering the subject group based on the content of the biomarker candidate substance selected in (a).

[3-4] The selecting method according to any of [3-1] to [3-3], further comprising (c) selecting a biomarker candidate substance selected in (a) as a biomarker candidate substance when the biomarker candidate substance selected in (a) is capable of discriminating a subject with high reliability in comparison with a result of clustering in (b).

[3-5] The selecting method according to [3-4], comprising, in (c),

(d) comparing a result of discriminating the subject using each of a series of any threshold candidate values of the biomarker candidate substance selected in (a) with the result of clustering in (b), thereby acquiring a sensitivity and a specificity of the discrimination using each threshold candidate value of the biomarker candidate substance;
(e) acquiring a reliability based on the sensitivity and the specificity acquired in (d); and
(f) determining whether or not the biomarker candidate substance is capable of discriminating the subject with high reliability.

[3-6] The selecting method according to any of [3-1] to [3-5], wherein the subject is suffering from PAH.

[3-7] The selecting method according to any of [3-1] to [3-6], wherein the sample is whole blood, plasma, or serum.

[3-8] The selecting method according to any of [3-1] to [3-7], wherein the vector similarity is a cosine similarity.

[3-9] The selecting method according to any of [3-1] to [3-8], wherein the biomarker candidate substance is selected based on the vector similarity of about 0.6 or more.

[3-10] The selecting method according to any of [3-1] to [3-9], wherein the subject group is clustered by K-medoids method.

[3-11] The selecting method according to any of [3-1] to [3-10], wherein the subject group is clustered into three clusters.

[3-12] The selecting method according to any of [3-4] to [3-11], wherein the reliability is indicated by AUC of a ROC curve acquired based on the sensitivity and the specificity.

[3-13] The selecting method according to [3-12], wherein the biomarker candidate substance is determined to be capable of discriminating the subject with high reliability when the AUC is about 0.8 or more.

[3-14] The selecting method according to any of [3-1] to [3-13], which is a computer-implemented method.

[3-15] The selecting method according to any of [3-2] to [3-14], wherein the IL-6 signaling pathway inhibitor is an antibody having an IL-6 signaling pathway inhibitory function, or an antigen-binding fragment thereof.

[3-16] The selecting method according to [3-15], wherein the antibody or an antigen-binding fragment thereof is an anti-IL-6 receptor antibody or an antigen-binding fragment thereof.

[4-1] A device for selecting a biomarker for predicting a content of IL-6 in a subject, comprising:

(a) a selection unit that selects a biomarker candidate substance based on a vector similarity acquired using a content of IL-6 and a content of the biomarker candidate substance in a sample collected from a subject group; and
(b) a clustering unit that clusters the subject group based on the content of the biomarker candidate substance selected in (a).

[4-2] A device for selecting a biomarker for predicting a responsiveness of a subject to a treatment with an IL-6 signaling pathway inhibitor, comprising:

(a) a selection unit that selects a biomarker candidate substance based on a vector similarity acquired using a content of IL-6 and a content of the biomarker candidate substance in a sample collected from a subject group; and
(b) a clustering unit that clusters the subject group based on the content of the biomarker candidate substance selected in (a).

[4-3] A device for selecting a biomarker for selecting a subject who is likely to have a responsiveness to a treatment with an IL-6 signaling pathway inhibitor, comprising:

(a) a selection unit that selects a biomarker candidate substance based on a vector similarity acquired using a content of IL-6 and a content of the biomarker candidate substance in a sample collected from a subject group; and
(b) a clustering unit that clusters the subject group based on the content of the biomarker candidate substance selected in (a).

[4-4] The selecting device according to any of [4-1] to [4-3], further comprising (c) a selection unit that selects a biomarker candidate substance selected in (a) as a biomarker candidate substance when the biomarker candidate substance selected in (a) is capable of discriminating a subject with high reliability in comparison with a result of clustering in (b).
[4-5] The selecting device according to [4-4], wherein the selection unit (c) comprises:

(d) an acquisition unit that compares a result of discriminating the subject using each of a series of any threshold candidate values of the biomarker candidate substance selected in (a) with the result of clustering in (b), thereby acquiring a sensitivity and a specificity of the discrimination using each threshold candidate value of the biomarker candidate substance;
(e) an acquisition unit that acquires a reliability based on the sensitivity and the specificity acquired in (d); and
(f) a determination unit that determines whether or not the biomarker candidate substance is capable of discriminating the subject with high reliability.

[4-6] The selecting device according to any of [4-1] to [4-5], wherein the subject is suffering from PAH.
[4-7] The selecting device according to any of [4-1] to [4-6], wherein the sample is whole blood, plasma, or serum.

[4-8] The selecting device according to any of [4-1] to [4-7], wherein the vector similarity is a cosine similarity.
[4-9] The selecting device according to any of [4-1] to [4-8], wherein the biomarker candidate substance is selected based on the vector similarity of about 0.6 or more.
[4-10] The selecting device according to any of [4-1] to [4-9], wherein the subject group is clustered by K-medoids method.
[4-11] The selecting device according to any of [4-1] to [4-10], wherein the subject group is clustered into three clusters.
[4-12] The selecting device according to any of [4-4] to [4-11], wherein the reliability is indicated by AUC of a ROC curve acquired based on the sensitivity and the specificity.
[4-13] The selecting device according to [4-12], wherein the biomarker candidate substance is determined to be capable of discriminating the subject with high reliability when the AUC is about 0.8 or more.
[4-14] The selecting device according to any of [4-2] to [4-13], wherein the IL-6 signaling pathway inhibitor is an antibody having an IL-6 signaling pathway inhibitory function, or an antigen-binding fragment thereof.
[4-15] The selecting device according to [4-14], wherein the antibody or an antigen-binding fragment thereof is an anti-IL-6 receptor antibody or an antigen-binding fragment thereof.
[5-1] Use of a biomarker for predicting a content of IL-6 in a subject, the biomarker comprising one or more cytokines selected from the group consisting of CXCL9/MIG, CCL4/MIP-1$\beta$, CCL27/CTACK, IL-1$\beta$/IL-1F2, GDF-15, IL-4, G-CSF, CXCL10/IP-10/CRG-2, and IL-1$\alpha$/IL-1F1.
[5-2] The use of a biomarker according to [5-1], wherein the biomarker comprises IL-1$\beta$/IL-1F2 and/or IL-4.
[5-3] The use of a biomarker according to [5-1] or [5-2], wherein the cytokine is derived from blood, plasma, or serum of a subject.
[5-4] The use of a biomarker according to any of [5-1] to [5-3], wherein the subject is suffering from PAH.
[5-5] Use of a biomarker for predicting a responsiveness of a subject to a treatment with an IL-6 signaling pathway inhibitor, the biomarker comprising one or more cytokines selected from the group consisting of CXCL9/MIG, CCL4/MIP-1$\beta$, CCL27/CTACK, IL-1$\beta$/IL-1F2, GDF-15, IL-4, G-CSF, IL-6, CXCL10/IP-10/CRG-2, and IL-1$\alpha$/IL-1F1.
[5-6] Use of a biomarker for predicting a responsiveness of a subject to a treatment with an IL-6 signaling pathway inhibitor, the biomarker comprising a combination of IL-6 and one or more cytokines selected from the group consisting of CXCL9/MIG, CCL4/MIP-1$\beta$, CCL27/CTACK, IL-1$\beta$/IL-1F2, GDF-

15, IL-4, G-CSF, CXCL10/IP-10/CRG-2, and 1L-1α/IL-1F1.

[5-7] The use of a biomarker according to [5-6], wherein the biomarker comprises a combination of IL-6 and one or more cytokines selected from the group consisting of IL-1β/IL-1F2 and IL-4.

[5-8] The use of a biomarker according to [5-6], wherein the biomarker comprises IL-6, IL-1β/IL-1F2, and IL-4.

[5-9] The use of a biomarker according to any of [5-5] to [5-8], wherein the cytokine is derived from blood, plasma, or serum of a subject.

[5-10] The use of a biomarker according to any of [5-5] to [5-8], wherein the subject is suffering from PAH.

[5-11] The use of a biomarker according to any of [5-5] to [5-10], wherein the IL-6 signaling pathway inhibitor is an antibody having an IL-6 signaling pathway inhibitory function, or an antigen-binding fragment thereof.

[5-12] The use of a biomarker according to [5-11], wherein the antibody or an antigen-binding fragment thereof is an anti-IL-6 receptor antibody or an antigen-binding fragment thereof.

[5-13] Use of a biomarker for selecting a subject who is likely to have a responsiveness to a treatment with an IL-6 signaling pathway inhibitor, the biomarker comprising one or more cytokines selected from the group consisting of CXCL9/MIG, CCL4/MIP-1β, CCL27/CTACK, IL-1β/IL-1F2, GDF-15, IL-4, G-CSF, IL-6, CXCL10/IP-10/CRG-2, and IL-1α/IL-1F1.

[5-14] Use of a biomarker for selecting a subject who is likely to have a responsiveness to a treatment with an IL-6 signaling pathway inhibitor, the biomarker comprising a combination of IL-6 and one or more cytokines selected from the group consisting of CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1.

[5-15] The use of a biomarker according to [5-14], wherein the biomarker comprises a combination of IL-6 and one or more cytokines selected from the group consisting of IL-1β/IL-1F2 and IL-4.

[5-16] The use of a biomarker according to [5-14], wherein the biomarker comprises IL-6, IL-1β/IL-1F2, and IL-4.

[5-17] The use of a biomarker according to any of [5-13] to [5-16], wherein the cytokine is derived from blood, plasma, or serum of a subject.

[5-18] The use of a biomarker according to any of [5-13] to [5-17], wherein the subject is suffering from PAH.

[5-19] The use of a biomarker according to any of [5-13] to [5-18], wherein the IL-6 signaling pathway inhibitor is an antibody having an IL-6 signaling pathway inhibitory function, or an antigen-binding fragment thereof.

[5-20] The use of a biomarker according to [5-19], wherein the antibody or an antigen-binding fragment thereof is an anti-IL-6 receptor antibody or an antigen-binding fragment thereof.

[6-1] A biomarker for predicting a content of IL-6 in a subject, the biomarker comprising

one or more cytokines selected from the group consisting of CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1.

[6-2] The biomarker according to [6-1], comprising IL-1 β/IL-1F2 and/or IL-4.

[6-3] The biomarker according to [6-1] or [6-2], wherein the cytokine is derived from blood, plasma, or serum of a subject.

[6-4] The biomarker according to any of [6-1] to [6-3], wherein the subject is suffering from PAH.

[7-1] A combination of biomarkers for predicting a content of IL-6 in a subject, the combination comprising two or more cytokines selected from the group consisting of CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1.

[7-2] The combination of biomarkers according to [7-1], comprising IL-1β/IL-1F2 and IL-4.

[7-3] The combination of biomarkers according to [7-1] or [7-2], wherein the cytokine is derived from blood, plasma, or serum of a subject.

[7-4] The combination of biomarkers according to any of [7-1] to [7-3], wherein the subject is suffering from PAH.

[8-1] A biomarker for predicting a responsiveness of a subject to a treatment with an IL-6 signaling pathway inhibitor, the biomarker comprising

one or more cytokines selected from the group consisting of CXCL9/MIG, CCL4/MIP-1β, CCL27/CTACK, IL-1β/IL-1F2, GDF-15, IL-4, G-CSF, IL-6, CXCL10/IP-10/CRG-2, and IL-1α/IL-1F1.

[8-2] A biomarker for predicting a responsiveness of a subject to a treatment with an IL-6 signaling pathway inhibitor, the biomarker comprising

a combination of IL-6 and one or more cytokines selected from the group consisting of CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1.

[8-3] The biomarker according to [8-2], comprising a combination of IL-6 and one or more cytokines selected from the group consisting of IL-1β/IL-1F2 and IL-4.

[8-4] The biomarker according to [8-2], comprising IL-6, IL-1β/IL-1F2, and IL-4.

[8-5] The biomarker according to any of [8-1] to [8-4], wherein the cytokine is derived from blood, plasma, or serum of a subject.

[8-6] The biomarker according to any of [8-1] to [8-5], wherein the subject is suffering from PAH.

[8-7] The biomarker according to any of [8-1] to [8-6], wherein the IL-6 signaling pathway inhibitor is an antibody having an IL-6 signaling pathway inhibitory function, or an antigen-binding fragment thereof.

[8-8] The biomarker according to [8-7], wherein the antibody or an antigen-binding fragment thereof is an anti-IL-6 receptor antibody or an antigen-binding fragment thereof.

[9-1] A combination of biomarkers for predicting a responsiveness of a subject to a treatment with an IL-6 signaling pathway inhibitor, the combination comprising two or more cytokines selected from the group consisting of CXCL9/MIG, CCL4/MIP-1β, CCL27/CTACK, IL-1β/IL-1F2, GDF-15, IL-4, G-CSF, IL-6, CXCL10/IP-10/CRG-2, and IL-1α/IL-1F1.

[9-2] A combination of biomarkers for predicting a responsiveness of a subject to a treatment with an IL-6 signaling pathway inhibitor, the combination comprising IL-6 and one or more cytokines selected from the group consisting of CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1.

[9-3] The combination of biomarkers according to [9-2], comprising IL-6 and one or more cytokines selected from the group consisting of IL-1β/IL-1F2 and IL-4.

[9-4] The combination of biomarkers according to [9-2], comprising IL-6, IL-1β/1L-1F2, and IL-4.

[9-5] The combination of biomarkers according to any of [9-1] to [9-4], wherein the cytokine is derived from blood, plasma, or serum of a subject.

[9-6] The combination of biomarkers according to any of [9-1] to [9-5], wherein the subject is suffering from PAH.

[9-7] The combination of biomarkers according to any of [9-1] to [9-6], wherein the IL-6 signaling pathway inhibitor is an antibody having an IL-6 signaling pathway inhibitory function, or an antigen-binding fragment thereof.

[9-8] The combination of biomarkers according to [9-7], wherein the antibody or an antigen-binding fragment thereof is an anti-IL-6 receptor antibody or an antigen-binding fragment thereof.

[10-1] A biomarker for selecting a subject who is likely to have a responsiveness to a treatment with an IL-6 signaling pathway inhibitor, the biomarker comprising

one or more cytokines selected from the group consisting of CXCL9/MIG, CCL4/MIP-1β, CCL27/CTACK, IL-1β/IL-1F2, GDF-15, IL-4, G-CSF, IL-6, CXCL10/IP-10/CRG-2, and IL-1α/IL-1F1.

[10-2] A biomarker for selecting a subject who is likely to have a responsiveness to a treatment with an IL-6 signaling pathway inhibitor, the biomarker comprising

a combination of IL-6 and one or more cytokines selected from the group consisting of CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1.

[10-3] The biomarker according to [10-2], comprising a combination of IL-6 and one or more cytokines selected from the group consisting of IL-1β/IL-1F2 and IL-4.

[10-4] The biomarker according to [10-2], comprising IL-6, IL-1β/IL-1F2, and IL-4.

[10-5] The biomarker according to any of [10-1] to [10-4], wherein the cytokine is derived from blood, plasma, or serum of a subject.

[10-6] The biomarker according to any of [10-1] to [10-5], wherein the subject is suffering from PAH.

[10-7] The biomarker according to any of [10-1] to [10-6], wherein the IL-6 signaling pathway inhibitor is an antibody having an IL-6 signaling pathway inhibitory function, or an antigen-binding fragment thereof.

[10-8] The biomarker according to [10-7], wherein the antibody or an antigen-binding fragment thereof is an anti-IL-6 receptor antibody or an antigen-binding fragment thereof.

[11-1] A combination of biomarkers for selecting a subject who is likely to have a responsiveness to a treatment with an IL-6 signaling pathway inhibitor, the combination comprising two or more cytokines selected from the group consisting of CXCL9/MIG, CCL4/MIP-lp, CCL27/CTACK, IL-1β/IL-1F2, GDF-15, IL-4, G-CSF, IL-6, CXCL10/IP-10/CRG-2, and IL-1α/IL-1F1.

[11-2] A combination of biomarkers for selecting a subject who is likely to have a responsiveness to a treatment with an IL-6 signaling pathway inhibitor, the combination comprising IL-6 and one or more cytokines selected from the group consisting of CXCL9/MIG, CCL4/MIP-1β, CCL27/CTACK, IL-1β/IL-1F2, GDF-15, IL-4, G-CSF, CXCL10/IP-10/CRG-2, and IL-1oc%IL-1F1.

[11-3] The combination of biomarkers according to [11-2], comprising IL-6 and one or more cytokines selected from the group consisting of IL-1β/IL-1F2 and IL-4.

[11-4] The combination of biomarkers according to [11-2], comprising IL-6, IL-1β/IL-1F2, and IL-4.

[11-5] The combination of biomarkers according to any of [11-1] to [11-4], wherein the cytokine is derived from blood, plasma, or serum of a subject.

[11-6] The combination of biomarkers according to any of [11-1] to [11-5], wherein the subject is suffering from PAH.

[11-7] The combination of biomarkers according to any of [11-1] to [11-6], wherein the IL-6 signaling pathway inhibitor is an antibody having an IL-6 signaling pathway inhibitory function, or an antigen-binding fragment thereof.

[11-8] The combination of biomarkers according to [11-7], wherein the antibody or an antigen-binding fragment thereof is an anti-IL-6 receptor antibody or an antigen-binding fragment thereof.

[12-1] A method for predicting a content of IL-6 in a subject, comprising measuring a content of the biomarker according to [6-1] or [6-2] in a sample collected from the subject.
[12-2] The method according to [12-1], wherein the sample is blood, plasma, or serum.
[12-3] The method according to [12-1] or [12-2], wherein the subject is suffering from PAH.
[12-4] A method for predicting a responsiveness of a subject to a treatment with an IL-6 signaling pathway inhibitor, comprising:

measuring a content of the biomarker according to [8-1] to [8-4] in a sample collected from the subject;
determining, based on the content of the biomarker, whether or not the subject is likely to have a responsiveness to a treatment with an IL-6 signaling pathway inhibitor.

[12-5] The method according to [12-4], wherein the sample is blood, plasma, or serum.
[12-6] The method according to [12-4] or [12-5], wherein the subject is suffering from PAH.
[12-7] The method according to any of [12-4] to [12-6], wherein the IL-6 signaling pathway inhibitor is an antibody having an IL-6 signaling pathway inhibitory function, or an antigen-binding fragment thereof.
[12-8] The method according to [12-7], wherein the antibody or an antigen-binding fragment thereof is an anti-IL-6 receptor antibody or an antigen-binding fragment thereof.
[12-9] A method for selecting a subject who is likely to have a responsiveness to a treatment with an IL-6 signaling pathway inhibitor, comprising:

measuring a content of the biomarker according to [10-1] to [10-4] in a sample collected from the subject;
determining, based on the content of the biomarker, whether or not the subject is likely to have a responsiveness to a treatment with an IL-6 signaling pathway inhibitor.

[12-10] The method according to [12-9], further comprising selecting a subject who is determined as likely to have a responsiveness to a treatment with an IL-6 signaling pathway inhibitor.
[12-11] The method according to [12-9] or [12-10], wherein the sample is blood, plasma, or serum.
[12-12] The selecting method according to any of [12-9] to [12-11], wherein the subject is suffering from PAH.
[12-13] The method according to any of [12-9] to [12-12], wherein the IL-6 signaling pathway inhibitor is an antibody having an IL-6 signaling pathway inhibitory function, or an antigen-binding fragment thereof.
[12-14] The method according to [12-13], wherein the antibody or an antigen-binding fragment thereof is an anti-IL-6 receptor antibody or an antigen-binding fragment thereof.
[13-1] A kit used for predicting a content of IL-6 in a subject, comprising a means for measuring the biomarker according to [6-1] or [6-2].
[13-2] A kit used for predicting a responsiveness of a subject to a treatment with an IL-6 signaling pathway inhibitor, comprising a means for measuring the biomarker according to any of [8-1] to [8-4].
[13-3] A kit used for selecting a subject who is likely to have a responsiveness to a treatment with an IL-6 signaling pathway inhibitor, comprising a means for measuring the biomarker according to any of [10-1] to [10-4].
[13-4] The kit according to any of [13-1] to [13-3], comprising a means for measuring a biomarker in blood, plasma, or serum collected from a subject.
[13-5] The kit according to any of [13-1] to [13-4], wherein the subject is suffering from PAH.
[13-6] The kit according to any of [13-1] to [13-5], comprising an antibody that specifically binds to a biomarker.
[13-7] The kit according to any of [13-1] to [13-6], wherein the IL-6 signaling pathway inhibitor is an antibody having an IL-6 signaling pathway inhibitory function, or an antigen-binding fragment thereof.
[13-8] The kit according to [13-7], wherein the antibody or an antigen-binding fragment thereof is an anti-IL-6 receptor antibody or an antigen-binding fragment thereof.
[14-1] A medicament for treating PAH, comprising an IL-6 signaling pathway inhibitor, wherein the medicament is administered to a subject who is selected as a subject likely to have a responsiveness to a treatment with the IL-6 signaling pathway inhibitor by the selecting method according to any of [12-9] to [12-14].
[14-2] The medicament according to [14-1], wherein the IL-6 signaling pathway inhibitor is an antibody having an IL-6 signaling pathway inhibitory function, or an antigen-binding fragment thereof.
[14-3] The medicament according to [14-2], wherein the antibody or an antigen-binding fragment thereof is an anti-IL-6 receptor antibody or an antigen-binding fragment thereof.
[14-4] A medicament for treating PAH, comprising an IL-6 signaling pathway inhibitor.
[14-5] A medicament for treating PAH, comprising an IL-6 signaling pathway inhibitor, wherein the medicament is administered to a subject suffering from PAH who has a serum content of IL-6 of 2.73 (pg/mL) or more.
[14-6] The medicament according to [14-4] or [14-5], wherein the IL-6 signaling pathway inhibitor is an antibody having an IL-6 signaling pathway inhibitory function, or an antigen-binding fragment thereof.

[14-7] The medicament according to [14-6], wherein the antibody or an antigen-binding fragment thereof is an anti-IL-6 receptor antibody or an antigen-binding fragment thereof.

[14-8] The medicament according to [14-3] or [14-7], wherein the anti-IL-6 receptor antibody or an antigen-binding fragment thereof contains at least one of three hypervariable regions (CDRs) in a heavy chain variable region amino acid sequence having SEQ ID NO: 1 and/or at least one of three hypervariable regions (CDRs) in a light chain variable region amino acid sequence having SEQ ID NO: 2.

[14-9] The medicament according to [14-8], wherein the anti-IL-6 receptor antibody or antigen-binding fragment thereof contains a heavy chain containing an amino acid sequence of SEQ ID NO: 3 and a light chain containing an amino acid sequence of SEQ ID NO: 4.

[14-10] The medicament according to [14-9], wherein the anti-IL-6 receptor antibody or an antigen-binding fragment thereof is satralizumab or an antigen-binding fragment thereof.

[15-1] A method for treating PAH, comprising administering to a subject selected by the selecting method according to any of [12-9] to [12-14] as a subject who is likely to have a responsiveness to a treatment targeting an IL-6 related cell signaling system, a therapeutically effective amount of an IL-6 signaling pathway inhibitor.

[15-2] The treating method according to [15-1], wherein the IL-6 signaling pathway inhibitor is an antibody having an IL-6 signaling pathway inhibitory function, or an antigen-binding fragment thereof.

[15-3] The treating method according to [15-2], wherein the antibody or an antigen-binding fragment thereof is an anti-IL-6 receptor antibody or an antigen-binding fragment thereof.

[15-4] A method for treating PAH, comprising administering a therapeutically effective amount of an IL-6 signaling pathway inhibitor to a subject suffering from PAH in need of treatment.

[15-5] A method for treating PAH, comprising administering an IL-6 signaling pathway inhibitor to a subject suffering from PAH in need of treatment who has a serum content of IL-6 of 2.73 (pg/mL) or more.

[15-6] The treating method according to [15-4] or [15-5], wherein the IL-6 signaling pathway inhibitor is an antibody having an IL-6 signaling pathway inhibitory function, or an antigen-binding fragment thereof.

[15-7] The treating method according to [15-6], wherein the antibody or an antigen-binding fragment thereof is an anti-IL-6 receptor antibody or an antigen-binding fragment thereof.

[15-8] The treating method according to [15-3] or [15-7], wherein the anti-IL-6 receptor antibody or an antigen-binding fragment thereof contains at least one of three hypervariable regions (CDRs) in a heavy chain variable region amino acid sequence having SEQ ID NO: 1 and/or at least one of three hypervariable regions (CDRs) in a light chain variable region amino acid sequence having SEQ ID NO: 2.

[15-9] The treating method according to [15-8], wherein the anti-IL-6 receptor antibody or antigen-binding fragment thereof contains a heavy chain containing an amino acid sequence of SEQ ID NO: 3 and a light chain containing an amino acid sequence of SEQ ID NO: 4.

[15-10] The treating method according to [15-9], wherein the anti-IL-6 receptor antibody or an antigen-binding fragment thereof is satralizumab or an antigen-binding fragment thereof.

[16-1] An IL-6 signaling pathway inhibitor for use in treating PAH, which is administered to a subject who is selected as a subject likely to have a responsiveness to a treatment with the IL-6 signaling pathway inhibitor by the selecting method according to any of [12-9] to [12-14].

[16-2] The IL-6 signaling pathway inhibitor according to [16-1], which is an antibody having an IL-6 signaling pathway inhibitory function, or an antigen-binding fragment thereof.

[16-3] The IL-6 signaling pathway inhibitor according to [16-2], wherein the antibody or an antigen-binding fragment thereof is an anti-IL-6 receptor antibody or an antigen-binding fragment thereof.

[16-4] An IL-6 signaling pathway inhibitor for use in treating PAH.

[16-5] An IL-6 signaling pathway inhibitor for use in treating PAH in a subject suffering from PAH who has a serum content of IL-6 of 2.73 (pg/mL) or more.

[16-6] The IL-6 signaling pathway inhibitor according to [16-4] or [16-5], which is an antibody having an IL-6 signaling pathway inhibitory function, or an antigen-binding fragment thereof.

[16-7] The IL-6 signaling pathway inhibitor according to [16-6], wherein the antibody or an antigen-binding fragment thereof is an anti-IL-6 receptor antibody or an antigen-binding fragment thereof.

[16-8] The IL-6 signaling pathway inhibitor according to [16-3] or [16-7], wherein the anti-IL-6 receptor antibody or an antigen-binding fragment thereof contains at least one of three hypervariable regions (CDRs) in a heavy chain variable region amino acid sequence having SEQ ID NO: 1 and/or at least one of three hypervariable regions (CDRs) in a light chain variable region amino acid sequence having SEQ ID NO: 2.

[16-9] The IL-6 signaling pathway inhibitor according to [16-8], wherein the anti-IL-6 receptor antibody or antigen-binding fragment thereof contains a heavy chain containing an amino acid sequence of SEQ ID NO: 3 and a light chain containing an amino acid sequence of SEQ ID NO: 4.

[16-10] The IL-6 signaling pathway inhibitor according to [16-9], wherein the anti-IL-6 receptor antibody or an antigen-binding fragment thereof is satralizumab or an antigen-binding fragment thereof.

[17-1] Use of an IL-6 signaling pathway inhibitor in producing of a medicament for treating PAH, wherein the medicament is administered to a subject who is selected as a subject who is likely to have a responsiveness to a treatment with the IL-6 signaling pathway inhibitor by the selecting method according to any of [12-9] to [12-14].

[17-2] The use according to [17-1], wherein the signaling pathway inhibitor is an antibody having an IL-6 signaling pathway inhibitory function, or an antigen-binding fragment thereof.

[17-3] The use according to [17-2], wherein the antibody or an antigen-binding fragment thereof is an anti-IL-6 receptor antibody or an antigen-binding fragment thereof.

[17-4] Use of an IL-6 signaling pathway inhibitor in producing of a medicament for treating PAH.

[17-5] Use of an IL-6 signaling pathway inhibitor in producing of a medicament for treating PAH, wherein the medicament is administered to a subject suffering from PAH who has a serum content of IL-6 of 2.73 (pg/mL) or more.

[17-6] The use according to [17-4] or [17-5], wherein the signaling pathway inhibitor is an antibody having an IL-6 signaling pathway inhibitory function, or an antigen-binding fragment thereof.

[17-7] The use according to [17-6], wherein the antibody or an antigen-binding fragment thereof is an anti-IL-6 receptor antibody or an antigen-binding fragment thereof.

[17-8] The use according to [17-3] or [17-7], wherein the anti-IL-6 receptor antibody or an antigen-binding fragment thereof contains at least one of three hypervariable regions (CDRs) in a heavy chain variable region amino acid sequence having SEQ ID NO: 1 and/or at least one of three hypervariable regions (CDRs) in a light chain variable region amino acid sequence having SEQ ID NO: 2.

[17-9] The use according to [17-8], wherein the anti-IL-6 receptor antibody or antigen-binding fragment thereof contains a heavy chain containing an amino acid sequence of SEQ ID NO: 3 and a light chain containing an amino acid sequence of SEQ ID NO: 4.

[17-10] The use according to [17-9], wherein the anti-IL-6 receptor antibody or an antigen-binding fragment thereof is satralizumab or an antigen-binding fragment thereof.

[18-1] A method for measuring a content of a biomarker, comprising

measuring a content of the biomarker according to any of [6-1] to [6-4], [8-1] to [8-8], and [10-1] to [10-8] in a sample collected from a subject.

[18-2] The measuring method according to [18-1], wherein the content of the biomarker is measured using an antibody that specifically binds to the biomarker.

[Advantageous Effects of Invention]

According to the present invention, a method for selecting a novel biomarker can be provided.

According to the present invention, a device for selecting a novel biomarker can be provided.

According to the present invention, a method for selecting a biomarker for predicting a content of IL-6 in a subject can be provided.

According to the present invention, a method for selecting a biomarker for predicting a responsiveness of a subject to a treatment with an IL-6 signaling pathway inhibitor can be provided.

According to the present invention, a method for selecting a biomarker for selecting a subject who is likely to have a responsiveness to a treatment with an IL-6 signaling pathway inhibitor can be provided.

According to the present invention, a device for selecting a biomarker for predicting a content of IL-6 in a subject can be provided.

According to the present invention, a device for selecting a biomarker for predicting a responsiveness of a subject to a treatment with an IL-6 signaling pathway inhibitor can be provided.

According to the present invention, a device for selecting a biomarker for selecting a subject who is likely to have a responsiveness to a treatment with an IL-6 signaling pathway inhibitor can be provided.

According to the present invention, use of a biomarker for predicting a content of IL-6 in a subject can be provided.

According to the present invention, use of a biomarker for predicting a responsiveness of a subject to a treatment with an IL-6 signaling pathway inhibitor can be provided.

According to the present invention, use of a biomarker for selecting a subject who is likely to have a responsiveness to a treatment with an IL-6 signaling pathway inhibitor can be provided.

According to the present invention, a biomarker for predicting a content of IL-6 in a subject can be provided.

According to the present invention, a biomarker for predicting a responsiveness of a subject to a treatment with an IL-6 signaling pathway inhibitor can be provided.

According to the present invention, a biomarker for selecting a subject who is likely to have a responsiveness to a treatment with an IL-6 signaling pathway inhibitor can be provided.

According to the present invention, a combination of biomarkers for predicting a content of IL-6 in a subject can be provided.

According to the present invention, a combination of biomarkers for predicting a responsiveness of a subject to a treatment with an IL-6 signaling pathway inhibitor can be provided.

According to the present invention, a combination of biomarkers for selecting a subject who is likely to have a responsiveness to a treatment with an IL-6 signaling pathway inhibitor can be provided.

According to the present invention, a method for predicting a content of IL-6 in a subject can be provided.

According to the present invention, a method for predicting a responsiveness of a subject to a treatment with an IL-6 signaling pathway inhibitor can be provided.

According to the present invention, a method for selecting a subject who is likely to have a responsiveness to a treatment with an IL-6 signaling pathway inhibitor can be provided.

According to the present invention, a kit used for predicting a content of IL-6 in a subject can be provided.

According to the present invention, a kit used for predicting a responsiveness of a subject to a treatment with an IL-6 signaling pathway inhibitor can be provided.

According to the present invention, a kit used for selecting a subject who is likely to have a responsiveness to a treatment with an IL-6 signaling pathway inhibitor can be provided.

According to the present invention, a medicament for treating PAH can be provided.

According to the present invention, a method for treating PAH can be provided.

According to the present invention, an IL-6 signaling pathway inhibitor for use in treating PAH can be provided.

According to the present invention, use of an IL-6 signaling pathway inhibitor in producing of a medicament for treating PAH can be provided.

According to the present invention, a method for measuring a content of a biomarker can be provided.

[Brief Description of Drawings]

[Figure 1] Figure 1 shows 49 cytokines measured in serum collected from 145 specimens in Example 1.
[Figure 2] Figure 2 shows a cosine similarity calculated for IL-6 and each of cytokines other than IL-6 (45 cytokines) in Example 2.
[Figure 3] Figure 3 shows a histogram created using the cosine similarity acquired for IL-6 and each of the cytokines other than IL-6 in Example 2.
[Figure 4] Figure 4 shows a network graph created based on the cosine similarity acquired for IL-6 and each of the cytokines other than IL-6 included in the IL-6 similar group in Example 2.
[Figure 5] Figure 5 shows a summary of the results of clustering content data in 145 specimens of the cytokines included in the IL-6 similar group into three clusters by k-medoids method in Example 2.
[Figure 6A-B] Figure 6A-B shows the results of graphing each cytokine located relatively close to IL-6 in the network graph of Figure 4, regarding the results of clustering the content data in 145 specimens of the cytokines included in the IL-6 similar group into three clusters by the k-medoids method in Example 2.
[Figure 7A-B] Figure 7A-B shows the results of graphing each cytokine located relatively close to IL-1β in the network graph of Figure 4, regarding the results of clustering the content data in 145 specimens of the cytokines included in the IL-6 similar group into three clusters by the k-medoids method in Example 2.
[Figure 8] Figure 8 shows a sensitivity and a specificity calculated by comparing the result of discriminating the subject with a threshold candidate value of the content of IL-6 and each threshold candidate value with the result of clustering by the k-medoids method in Example 3.
[Figure 9] Figure 9 shows a sensitivity and a specificity calculated by comparing the result of discriminating the subject with a threshold candidate value of the content of IL-1β and each threshold candidate value with the result of clustering by the k-medoids method in Example 3.
[Figure 10] Figure 10 shows the reliability (AUC) of each cytokine included in the IL-6 similar group obtained in Example 3.

[Description of Embodiments]

Hereinafter, embodiments of the present invention will be described in detail. The present invention is not limited to the following embodiments, and can be carried out with appropriate modifications within the extent that the effects of the present invention are not impaired.

= = Explanation of terms = =

[Biomarker]

As used herein, the term "biomarker" refers to, unless otherwise specified, a substance derived from a subject and/or data derived from a subject that reflects a state of the subject or a change thereof and would be an indicator of the state of the subject.

[Responsiveness]

As used herein, the term "responsiveness" is, unless otherwise specified, synonymous with the term therapeutic susceptibility and is not limited as long as it is a state in which any favorable outcome is consecutively or temporarily obtained due to a treatment of a disease in a subject. Examples of the "favorable outcome" include suppression or improvement of a systemic symptom in a subject, suppression or improvement of pathological symptoms, suppression or improvement of test data reflecting a disease condition, and/or improvement of prognosis of a subject.

[IL-6-related disease]

As used herein, the term "interleukin-6 (IL-6)-related disease" encompasses, unless otherwise specified, a disease in which at least a part of direct or indirect causes of onset or development of the disease is related to IL-6. Examples of the IL-6-related disease include rheumatoid arthritis, juvenile idiopathic arthritis, systemic juvenile idiopathic arthritis, Castleman disease, systemic lupus erythematosus (SLE), lupus nephritis, Crohn's disease, lymphoma, ulcerative colitis, anemia, vasculitis, Kawasaki disease, Still disease, amyloidosis, multiple sclerosis, transplantation, age-related macular degeneration, ankylosing spondylitis, psoriasis, psoriatic arthritis, chronic obstructive pulmonary disease (COPD), IgA nephropathy, osteoarthritis, asthma, diabetic nephropathy, graft-versus host disease (GVHD), endometriosis, hepatitis (NASH), myocardial infarction, arteriosis, cepsis, osteopathy, diabetes, multiple myeloma, prostate cancer, renal cancer, B-cell non-Hodgkin's lymphoma, pancreatic cancer, lung cancer, esophageal cancer, colorectal cancer, cancer cachexia, cancer neuroinvasion, myopic choroid angiogenesis, idiopathic choroidal neovascularization, uveitis, chronic thyroiditis, delayed hypersensitivity, contact dermatitis, atopic dermatitis, mesothelioma, multiple myositis, dermatomyositis, pan-uveitis, frontal uveitis, intermediate uveitis, scleritis, keratitis, orthitis, optic neuritis, diabetic retinitis, proliferative retinitis, dry eye, postoperative inflammation, neuromyelitis optica, myasthenia gravis, and pulmonary hypertension.

[IL-6 signaling pathway inhibitor]

The biological activity signals of IL-6 are transmitted by IL-6 that binds to an IL-6 receptor on the cell membrane to form an IL-6/IL-6 receptor complex, which then binds to gp130. Thus, unless otherwise specified, the "IL-6 signaling pathway inhibitor" as used herein may be any molecule capable of neutralizing, blocking, inhibiting, suppressing, reducing, or interfering with at least a part of the function or activity of IL-6, an IL-6 receptor, and/or gp130. For example, the IL-6 signaling pathway inhibitor may be an antibody, an antibody fragment, a binding polypeptide, a peptide, or a non-peptide molecule.

In one embodiment, the IL-6 signaling pathway inhibitor may be an anti-IL-6 antibody, an anti-IL-6 receptor antibody, and/or an anti-gp130 antibody, or an IL-6 antagonist compound.

In one embodiment, the IL-6 signaling pathway inhibitor is preferably an antibody having an IL-6 signaling pathway inhibitory function, or an antigen-binding fragment thereof, such as an anti-IL-6 antibody or antigen-binding fragment thereof, or an anti-IL-6 receptor antibody or antigen-binding fragment thereof, and more preferably an anti-IL-6 receptor antibody or an antigen-binding fragment thereof.

In one embodiment, examples of the anti-IL-6 receptor include an antibody that contains at least one of three hypervariable regions (CDRs) in a heavy chain variable region amino acid sequence having SEQ ID NO: 1 and/or at least one of three hypervariable regions (CDRs) in a light chain variable region amino acid sequence having SEQ ID NO: 2.

In one embodiment, examples of the anti-IL-6 receptor include an antibody that contains a heavy chain containing an amino acid sequence of SEQ ID NO: 3 and a light chain containing an amino acid sequence of SEQ ID NO: 4.

In one embodiment, examples of the anti-IL-6 receptor antibody include satralizumab, tocilizumab, and/or sarilumab, but satralizumab and/or tocilizumab are preferred, and satralizumab is more preferred.

The antibody may be a chimeric antibody, a humanized antibody, a human antibody, or an antibody derived from a library, or an antibody fragment thereof. The antigen-binding fragment encompasses an antibody fragment that is missing a portion of the full-length antibody while maintaining the antigen-binding ability. Examples thereof include Fab, Fab', F(ab')$_2$, and Fv. Furthermore, specific examples of a low molecular weight antibody include Fab, Fab', F(ab')$_2$, Fv, scFv, diabody, and sc(Fv)$_2$.

The antibody can be obtained as a polyclonal antibody or a monoclonal antibody by use of a known means in the art. The origin of the antibody is not particularly limited, but preferred examples include an antibody derived from a mammal, and more preferably an antibody derived from a human. Examples of a monoclonal antibody derived from a mammal include those produced by a hybridoma and those produced by a host that has been transformed with an expression vector containing an antibody gene by a genetic engineering approach.

The antibody or an antigen-binding fragment thereof may be a conjugated antibody that is conjugated to any molecule such as polyethylene glycol (PEG), a radioactive material, a toxin, or a therapeutic compound.

The antibody includes not only a bivalent antibody represented by IgG, but also a monovalent antibody or a multivalent antibody represented by IgM. The multivalent antibody according to the present invention includes a multivalent antibody having all the same antigen binding sites, or a multivalent antibody having partly or all different antigen binding sites.

The antibody may be a bispecific antibody. The bispecific antibody refers to an antibody having a variable region that recognizes different epitopes in the same antibody molecule, where the epitopes may be present in different molecules or may be present in the same molecule.

The bispecific antibody can be produced by known methods. For example, a bispecific antibody can be produced by combining two types of antibodies whose recognition antigens are different. The antibodies to be combined may be 1/2 molecules of an antibody which have an H chain and an L chain, respectively, or may be 1/4 molecules of an antibody which consist of an H chain only. Alternatively, hybridomas producing different monoclonal antibodies may be fused to create bispecific antibody-producing fusion cells. Furthermore, the bispecific antibody can be produced by genetic engineering techniques.

= = Method for selecting novel biomarker = =

The method for selecting a biomarker according to the present embodiments is a method for selecting a novel biomarker, comprising:

(a) selecting a biomarker candidate substance based on a vector similarity acquired using a content of a reference biomarker and a content of the biomarker candidate substance in a sample collected from a subject group; and
(b) clustering the subject group based on the content of the biomarker candidate substance selected in (a).

In one embodiment, the method for selecting a novel biomarker is the selecting method further comprising (c) selecting a biomarker candidate substance selected in (a) as a biomarker candidate substance when the biomarker candidate substance selected in (a) is capable of discriminating a subject with high reliability in comparison with a result of clustering in (b).

In one embodiment, the method for selecting a novel biomarker is the selecting method comprising, in (c),

(d) comparing a result of discriminating the subject using each of a series of any threshold candidate values of the biomarker candidate substance selected in (a) with the result of clustering in (b), thereby acquiring a sensitivity and a specificity of the discrimination using each threshold candidate value of the biomarker candidate substance;
(e) acquiring a reliability based on the sensitivity and the specificity acquired in (d); and
(f) determining whether or not the biomarker candidate substance is capable of discriminating the subject with high reliability.

[Subject group]

The "subject" in the method for selecting a novel biomarker is not limited as long as it is an organism in which the reference biomarker is present in vivo on a constant or temporary basis. For example, the subject may be a mammal or an animal other than a mammal. The mammal may be a human or a non-human animal, and the non-human animal species may be, for example, a monkey, a dog, a cat, a horse, a cow, a pig, a sheep, a goat, a rabbit, a guinea pig, a hamster, a mouse, and/or a rat. The subject is not limited by its use such as a domestic animal, a pet animal, a laboratory animal, or the like, but is preferably a mammal, and more preferably a human.

The subject group is a group including a number of subjects capable of clustering in (b) and is a group including at least two or more subjects. For example, when clustering a subject group into three clusters, the subject group includes at least three subjects.

The subject group may be a group of subjects extracted based on a state of the subject, a group of randomly selected subjects, or a group including all subjects available when performing the method for selecting a novel biomarker. The subject group can be appropriately determined by those skilled in the art based on what kind of biomarker is intended to be selected by the method for selecting a novel biomarker.

Here, the state of the subject can be any state of the subject, for example, may be a state related to a particular disease or an unrelated state.

When the state of the subject is a state related to a particular disease, the state of the subject is not limited as long as the state is at least partially related to the particular disease. Examples of the state related to a particular disease include a state in which a subject is suspected of suffering from a particular disease, a state in which whether or not a subject is suffering from a particular disease, a state in which whether or not a subject is suffering from any subtype of a particular disease, a state in which whether or not a subject suffering from a particular disease is responsive to a particular treatment, a state of progression of the particular disease which the subject is suffering from, and/or a state of improvement of a symptom of a particular disease which the subject is suffering from.

When the state of the subject is a state unrelated to a particular disease, examples of the state of the subject include physiological characteristics, genetic conditions such as sex or race, age, and/or geographical conditions such as place of residence and area of activity, which are unrelated to any disease.

[Reference biomarker]

In one embodiment, the reference biomarker in the method for selecting a novel biomarker may be a substance that is unknown to be related to the state of a subject or may be a substance that is already known to reflect a state of interest, i.e., a substance that is known to be useful as an indicator of the state of a subject.

In one embodiment, when the reference biomarker is a substance that is unknown to be related to the state of a subject, the method for selecting a novel biomarker can be used for selecting a novel biomarker for predicting a content of the reference biomarker in the subject, or the like.

In another embodiment, when the reference biomarker is a substance that is already known to be useful as an indicator of the state of the subject, the method for selecting a novel biomarker can be used, for example, for acquiring a biomarker for predicting a content of the reference biomarker in a subject and/or for acquiring a novel biomarker that can indicate, in combination with or in place of the reference biomarker, the state of the subject for which the reference biomarker is known to be useful as an indicator.

In this case, the method for selecting a novel biomarker can be used, for example, for acquiring a novel marker that is better as an indicator than the reference biomarker, for acquiring a novel biomarker that allows for a more diversified and/or more reliable understanding of the state of a subject by using it in combination with the reference biomarker, and/or for acquiring a novel biomarker that is more economical, convenient, and/or versatile than the reference biomarker.

[Step (a)]

The method for selecting a novel biomarker comprises (a) selecting a biomarker candidate substance based on a vector similarity acquired using a content of a reference biomarker and a content of the biomarker candidate substance in a sample collected from a subject group.

Here, the sample is a biological sample collected from the subject group, and is not limited as long as it is a sample in which the contents of a reference biomarker and a biomarker candidate substance can be measured. For example, the sample may be a solid tissue sample obtained by a biopsy or during a treatment, or may be blood (whole blood), plasma, or serum.

The reference biomarker and the biomarker candidate substance may be derived from the same tissue or may be derived from different tissues in the same individual.

The contents of the reference biomarker and the biomarker candidate substance in the sample may be measured by a conventional method in the art based on what substance the biomarker is.

For example, when the reference biomarker and the biomarker candidate substance are cytokines, the contents can be measured by the method described in the section [Measurement of biomarker in sample] in "Method for measuring biomarker".

Here, the contents of the reference biomarker and the biomarker candidate substance may be an absolute value, a relative concentration, a weight per unit volume, raw data measured to know the absolute concentration, or the like.

In one embodiment, when plasma or serum is measured as a sample, it is preferred that the content is a weight per unit volume of the sample, such as 1 mL or 1 μL.

In one embodiment, the vector similarity is not limited to the extent it is typically used in the art to indicate a similarity of behaviors of a reference biomarker and a biomarker candidate substance based on the content of the reference biomarker and the content of the biomarker candidate substance. For example, the vector similarity may be a cosine similarity represented by the following formula, or may be a Euclidean distance.

$$\frac{\vec{x} \cdot \vec{y}}{|\vec{x}||\vec{y}|} = \frac{x_1 y_1 + x_2 y_2 + \cdots + x_n y_n}{\sqrt{x_1^2 + x_2^2 + \cdots + x_n^2} \sqrt{y_1^2 + y_2^2 + \cdots + y_n^2}}$$

In the above formula, x is the content of the reference biomarker and y is the content of the biomarker candidate substance (or vice versa). n is the number of subjects constituting the subject group.

The cosine similarity corresponds to cosθ when the angle between the vector of x and the vector of y is taken as θ. The closer the cosine similarity is to 1, the closer the vector of x and the vector of y are to the same direction. The vector similarity indicates a similarity of behaviors of a reference biomarker and each of biomarker candidate substances.

In one embodiment, the values of the content of the reference biomarker and the content of the biomarker candidate substance used to acquire the vector similarity may be preprocessed by a conventional method in the art.

In one embodiment, processing may be performed to exclude extremely low values and/or extremely high values. The method for such processing can be appropriately determined by those skilled in the art by considering the number of subjects constituting the subject group (the number of measured values), the distribution status of the content data, the purpose of carrying out the selecting method, and so on by a conventional method in the art.

For example, a processing may be performed by excluding the value detected as an abnormal value using any conventional anomaly detection method in the field of data analysis, or by performing a process to exclude extremely low values and/or extremely high values by excluding higher and/or lower values corresponding to any set ratios or numbers.

In a more specific embodiment, a processing may be performed by treating all content values indicating equal to or less than the measurement sensitivity as 0 (zero) to exclude extremely low values.

In one embodiment, for example, the top 30, top 20, top 10, or top 5 values may be taken as 30th, 20th, 10th, or 5th values from the top, respectively, to exclude extremely high values.

As the vector similarity, for example, in the case of cosine similarity, is closer to 1, the closer the vector of x and the vector of y are to the same direction, as described above. In other words, when the cosine similarity is 1, the reference biomarker and the biomarker candidate substance exhibit the same behavior, and the closer the cosine similarity is to 1, they exhibit the higher similarity behavior.

The vector similarity that will serve as the basis for selecting a biomarker candidate substance may be appropriately set by those skilled in the art using a conventional method in the art, with consideration of the purpose of carrying out the method for selecting a novel biomarker and the subsequent step. It is preferred, however, considering the acquisition of an effective novel biomarker that can predict the content of a reference biomarker, the vector similarity is set to about 0.5 or more, about 0.6 or more, about 0.7 or more, about 0.8 or more, or about 0.9 or more, and more preferably set to about 0.6 or more.

[Step (b)]

The method for selecting a novel biomarker comprises (b) clustering the subject group based on the content of the biomarker candidate substance selected in (a).

In one embodiment, clustering is preferably a classification by unsupervised machine learning for the data of the content of all the biomarker candidate substances selected in (a).

In one embodiment of this case, the clustering may be, for example, a clustering by a hierarchical clustering method or a non-hierarchical clustering method such as k-means method or k-medoids method, and is not limited to the extent that the desired clustering is possible. It is preferred, however, that the clustering is a clustering by a non-hierarchical clustering method, more preferably a clustering by k-medoids method.

The number of clusters of a subject group can be determined by those skilled in the art according to a conventional method in the art, and may be clustered into two clusters, three clusters, or four clusters.

In one embodiment, when clustering is performed, for example, using the k-means method or the k-medoids method, the number of clusters needs to be set at the time of performing.

[Step (c)]

In one embodiment, it is preferred that the method for selecting a novel biomarker further comprises (c) selecting a biomarker candidate substance selected in (a) as a biomarker candidate substance when the biomarker candidate substance selected in (a) is capable of discriminating a subject with high reliability in comparison with a result of clustering in (b).

In one embodiment, the method for selecting a novel biomarker may be the selecting method comprising, in (c),

(d) comparing a result of discriminating the subject using each of a series of any threshold candidate values of the biomarker candidate substance selected in (a) with the result of clustering in (b), thereby acquiring a sensitivity and a specificity of the discrimination using each threshold candidate value of the biomarker candidate substance;

(e) acquiring a reliability based on the sensitivity and the specificity acquired in (d); and

(f) determining whether or not the biomarker candidate substance is capable of discriminating the subject with high reliability.

In one embodiment, the reliability is preferably indicated by an area under the curve (AUC) of a receiver operating curve (ROC) acquired based on sensitivity and specificity.

In one embodiment, in (f), it is determined that the biomarker candidate is capable of discriminating the subject with high reliability, preferably when the AUC is about 0.7 or more, about 0.8 or more, or about 0.9 or more, and more preferably when the AUC is about 0.8 or more.

In one embodiment, the method for selecting a novel biomarker may be a computer-implemented method implemented by a computer.

Herein, an embodiment of (c) comprising (d) to (f), will be described, for example, assuming that the subject group is clustered into three clusters based on the content of the biomarker candidate substance in (b).

Of the three clusters obtained by clustering, the cluster having a high content of the biomarker candidate substance (including one or two clusters) can be set as the cluster of the "subject having a high content of the reference biomarker", and the other cluster can be set as the cluster of the "subject having a low content of the reference biomarker", which is a "result of clustering in (b)".

In (d), the "sensitivity" and the "specificity" of the "biomarker candidate substance selected in (a)" can be acquired, for example, as follows.

First, for the content of a certain biomarker candidate substance selected in (a), a series of any threshold candidate values is set, and each subject is discriminated whether the subject is a "subject having a high content of the reference biomarker" whose content is greater than or equal to the threshold candidate value (or greater than the threshold candidate value), or a "subject having a low content of the reference biomarker" whose content is less than the threshold candidate value (or less than or equal to the threshold candidate value). Then, by comparing the result of discriminating the "subject having a high content of the reference biomarker" and the "subject having a low content of the reference biomarker" using each threshold candidate value with the results of clustering the "subject having a high content of the reference biomarker" and the "subject having a low content of the reference biomarker" (the result of clustering in (b)), the sensitivity and the specificity of the discrimination of "subject having a high content of the reference biomarker" and "subject having a low content of the reference biomarker" can be acquired in the case where each threshold candidate value is used.

More specifically, by comparing the result of discriminating the "subject having a high content of the reference biomarker" by a certain threshold candidate value with the result of clustering the "subject having a high content of the reference biomarker" (the result of clustering in (b), the probability (sensitivity) that the "subject having a high content of the reference biomarker" can be correctly discriminated by the threshold candidate value can be obtained. Furthermore, by comparing the result of discriminating the "subject having a low content of the reference biomarker" by a certain threshold candidate value with the result of clustering the "subject having a low content of the reference biomarker" (the result of clustering in (b)), the probability (specificity) that the "subject having a low content of the reference biomarker" can be correctly discriminated by the threshold candidate value can be obtained.

This method is repeated to obtain a sensitivity and a specificity corresponding to each of the series of any threshold candidate values.

In (e), a reliability is acquired based on the sensitivity and the specificity acquired in (d). The reliability can be indicated, for example, by the area under the curve (AUC) of the ROC curve drawn based on the sensitivity and the specificity.

In other words, the AUC thus acquired shows the reliability of the result of discriminating using the biomarker candidate substance to discriminate the subject compared with the result of clustering at (b).

In (f), whether or not the biomarker candidate substance is capable of discriminating a subject with high reliability is determined. This determination is preferably based on the reliability indicated by the AUC as described above. Then, when the AUC is about 0.7 or more, about 0.8 or more, or about 0.9 or more, more preferably when the AUC is about 0.8 or more, it is discriminated that the biomarker candidate is capable of discriminating the subject with high reliability.

With the above-described method for selecting a novel biomarker, it is possible to select a novel biomarker that is effective for predicting a content of a reference biomarker in a sample acquired from a subject.

= = Device for selecting novel biomarker = =

The device for selecting a novel biomarker according to the present embodiment is a device for carrying out the method described in the section "Method for selecting novel biomarker".

That is, the device for selecting a novel biomarker according to the present embodiments is a device for selecting a novel biomarker, comprising:

(a) a selection unit that selects a biomarker candidate substance based on a vector similarity acquired using a content of a reference biomarker and a content of the biomarker candidate substance in a sample collected from a subject group; and
(b) a clustering unit that clusters the subject group based on the content of the biomarker candidate substance selected in (a).

In one embodiment, the device for selecting a novel biomarker is the selecting device further comprising (c) a selection unit that selects a biomarker candidate substance selected in (a) as a biomarker candidate substance when the biomarker candidate substance selected in (a) is capable of discriminating a subject with high reliability in comparison with a result of clustering in (b).

In one embodiment, the device for selecting a novel biomarker is the selecting device, wherein the selection unit (c) comprises:

(d) an acquisition unit that compares a result of discriminating the subject using each of a series of any threshold candidate values of the biomarker candidate substance selected in (a) with the result of clustering in (b), thereby acquiring a sensitivity and a specificity of the discrimination using each threshold candidate value of the biomarker candidate substance;
(e) an acquisition unit that acquires a reliability based on the sensitivity and the specificity acquired in (d); and
(f) a determination unit that determines whether or not the biomarker candidate substance is capable of discriminating the subject with high reliability.

In the device for selecting a novel biomarker, each embodiment and a preferred embodiment of the "subject group", the "reference biomarker" and the like and in the steps (a) to (f) are as described in the section "Method for selecting novel biomarker".

= = Method for selecting biomarker for predicting content of IL-6 = =

The method for selecting a biomarker for predicting a content of IL-6 in a subject according to the present embodiment is
a selecting method comprising:

(a) selecting a biomarker candidate substance based on a vector similarity acquired using a content of IL-6 and a content of the biomarker candidate substance in a sample collected from a subject group; and
(b) clustering the subject group based on the content of the biomarker candidate substance selected in (a).

In one embodiment, the method for selecting a biomarker for predicting a content of IL-6 is
a selecting method, further comprising (c) selecting a biomarker candidate substance selected in (a) as a biomarker candidate substance when the biomarker candidate substance selected in (a) is capable of discriminating a subject with high reliability in comparison with a result of clustering in (b).

In one embodiment, the method for selecting a biomarker for predicting a content of IL-6 is the selecting method comprising, in (c),

(d) comparing a result of discriminating the subject using each of a series of any threshold candidate values of the biomarker candidate substance selected in (a) with the result of clustering in (b), thereby acquiring a sensitivity and a specificity of the discrimination using each threshold candidate value of the biomarker candidate substance;
(e) acquiring a reliability based on the sensitivity and the specificity acquired in (d); and
(f) determining whether or not the biomarker candidate substance is capable of discriminating the subject with high reliability.

[Subject group]

The "subject" in the method for selecting a biomarker for predicting a content of IL-6 is not limited as long as it is an organism in which IL-6 is present in vivo on a constant or temporary basis. For example, the subject may be a mammal or an animal other than a mammal. The mammal may be a human or a non-human animal, and the non-human animal species may be, for example, a monkey, a dog, a cat, a horse, a cow, a pig, a sheep, a goat, a rabbit, a guinea pig, a hamster, a mouse, and/or a rat. The subject is not limited by its use such as a domestic animal, a pet animal, or a laboratory animal, but is preferably a mammal, and more preferably a human.

The subject group is a group including a number of subjects capable of clustering in (b), and is a group including at least two or more subjects. For example, when clustering a subject group into three clusters, the subject group includes at least three subjects.

The subject group may be a group of subjects extracted based on a state of the subject, a group of randomly selected subjects, or a group including all subjects available when performing the method for selecting a biomarker for predicting a content of IL-6. The subject group can be appropriately determined by those skilled in the art based on what kind of biomarker is intended to be selected by the method for selecting a biomarker for predicting a content of IL-6.

Here, the state of the subject can be any state of the subject, for example, may be a state related to a particular disease or an unrelated state.

When the state of the subject is a state related to a particular disease, the state of the subject is not limited as long as the state is at least partially related to the particular disease. Examples of the state related to a particular disease include a state in which a subject is suspected of suffering from a particular disease, a state in which whether or not a subject is suffering from a particular disease, a state in which whether or not a subject is suffering from any subtype of a particular disease, a state in which whether or not a subject suffering from a particular disease is responsive to a certain treatment, a state of progression of the particular disease which the subject is suffering from, and/or a state of improvement of a symptom of a particular disease which the subject is suffering from.

When the state of the subject is a state unrelated to a particular disease, examples of the state of the subject include physiological characteristics, genetic conditions such as sex or race, age, and/or geographical conditions such as place of residence and area of activity, which are unrelated to any disease.

[IL-6-related disease]

In one embodiment, the subject group in the method for selecting a biomarker for predicting a content of IL-6 is preferably a subject group suspected of suffering from an IL-6-related disease or a subject group suffering from an IL-6-related disease. Here, the "IL-6-related disease" is as described in the section "Explanation of terms".

In one embodiment, an IL-6-related disease may be a disease in which the IL-6 content or the presence or absence of IL-6 in a sample changes by whether or not the subject is related to the disease, or a disease in which the content of IL-6 or the presence or absence of IL-6 in a sample changes based on the state of each subject in a subject group suffering from the IL-6-related disease, and the state of the subject group can be classified into subtypes based on such changes.

In one embodiment, a preferred example of the IL-6-related disease is pulmonary hypertension. In other words, the subject group is preferably a subject group suffering from pulmonary hypertension.

Here, pulmonary hypertension is clinically classified into pulmonary arterial hypertension (PAH, group 1), pulmonary hypertension with left cardiac heart disease (group 2), pulmonary hypertension with pulmonary disease and/or hypoxemia (group 3), CTEPH (group 4), and pulmonary hypertension with an unspecified multifactorial mechanism (group 5) (Guidelines for Treatment of Pulmonary Hypertension (revised version in 2017)).

In one embodiment, the pulmonary hypertension is more preferably pulmonary arterial hypertension (PAH). In other words, the subject group is preferably a subject group suffering from PAH.

It has been reported that IL-6 in blood is elevated and expression of IL-6 receptors in diseased organs is high in PAH, and that treatments with IL-6 signaling pathway inhibitors are effective in PAH. That is, it is recognized that PAH can be treated by inhibition of IL-6 action, and the blood content of IL-6 in a subject suffering from PAH would be an indicator of responsiveness to a treatment with an IL-6 signaling pathway inhibitor.

Thus, in one embodiment, when the subject group in the method for selecting a biomarker for predicting a content of IL-6 is a subject group suffering from PAH, the selecting method can be used for the purpose of acquiring a biomarker for predicting a content of IL-6 and/or for the purpose of acquiring a biomarker that can indicate a responsiveness of a subject to a treatment with an IL-6 signaling pathway inhibitor in combination with IL-6 or in place of IL-6. Here, the "IL-6 signaling pathway inhibitor" is as described in the section "Explanation of terms".

[Step (a)]

The method for selecting a biomarker for predicting a content of IL-6 comprises selecting a biomarker candidate substance based on a vector similarity acquired using a content of IL-6 and a content of the biomarker candidate substance in a sample collected from a subject group.

Here, the sample is a biological sample collected from the subject group, and is not limited as long as it is a sample in which the contents of IL-6 and a biomarker candidate substance can be measured. For example, the sample may be a solid tissue sample obtained by a biopsy or during a treatment, or may be blood (whole blood), plasma, or serum, and is preferably blood (whole blood), plasma, or serum.

IL-6 and the biomarker candidate substance may be derived from the same tissue or may be derived from different tissues in the same individual, but is preferably derived from the same tissue in the same individual, that is, preferably derived from blood (whole blood), plasma, or serum in the same individual.

The contents of IL-6 and the biomarker candidate substance in a sample may be measured by a conventional method in the art. For example, the contents can be measured by the method described in the section [Measurement of biomarker in sample] in "Method for measuring biomarker".

Here, the contents of IL-6 and the biomarker candidate substance may be an absolute value, a relative concentration, a weight per unit volume, raw data measured to know the absolute concentration, or the like.

In one embodiment, when plasma or serum is measured as a sample, it is preferred that the content is a weight per unit volume of the sample, such as 1 mL or 1 μL.

In one embodiment, the vector similarity is not limited to the extent it indicates a similarity of behaviors of IL-6 and a biomarker candidate substance based on the content of IL-6 and the content of the biomarker candidate substance. For example, the vector similarity may be a cosine similarity represented by the following formula, or may be a Euclidean distance, but preferably a cosine similarity.

$$\frac{\vec{x} \cdot \vec{y}}{|\vec{x}||\vec{y}|} = \frac{x_1 y_1 + x_2 y_2 + \cdots + x_n y_n}{\sqrt{x_1^2 + x_2^2 + \cdots + x_n^2}\sqrt{y_1^2 + y_2^2 + \cdots + y_n^2}}$$

In the above formula, x is the content of IL-6 and y is the content of the biomarker candidate substance (or vice versa). n is the number of subjects constituting the subject group.

The cosine similarity corresponds to cosθ when the angle between the vector of x and the vector of y is taken as θ. The closer the cosine similarity is to 1, the closer the vector of x and the vector of y are to the same direction. The vector similarity indicates a similarity of behaviors of IL-6 and each of the biomarker candidate substances.

In one embodiment, the values of the content of IL-6 and the content of the biomarker candidate substance used to acquire the vector similarity may be preprocessed by a conventional method in the art.

In one embodiment, processing may be performed to exclude extremely low values and/or extremely high values. The method for such processing can be appropriately determined by those skilled in the art by considering the number of subjects constituting the subject group (the number of measured values), the distribution status of the content data, the purpose of carrying out the selecting method, and so on by a conventional method in the art.

For example, a processing may be performed by excluding the value detected as an abnormal value using any conventional anomaly detection method in the field of data analysis, or by performing a process to exclude extremely low values and/or extremely high values by excluding higher and/or lower values corresponding to a predetermined ratio or number.

In a more specific embodiment, a processing may be performed by treating all measured content values indicating equal to or less than the measurement sensitivity as 0 (zero) to exclude extremely low values.

In one embodiment, for example, the top 30, top 20, top 10, or top 5 values may be taken as 30th, 20th, 10th, or 5th values from the top, respectively, to exclude extremely high values.

As described above, the closer the vector similarity, for example, in the case of cosine similarity, is to 1, the closer the vector of x and the vector of y are to the same direction. In other words, when the cosine similarity is 1, IL-6 and the biomarker candidate substance exhibit the same behavior, and the closer the cosine similarity is to 1, they exhibit the higher similarity behavior.

The vector similarity that will serve as the basis for selecting a biomarker candidate substance may be appropriately set by those skilled in the art using a conventional method in the art, with consideration of the purpose of performing the method for selecting a biomarker for predicting a content of IL-6 and the subsequent step.

It is preferred, however, considering the acquisition of an effective biomarker that can predict the content of IL-6, the vector similarity is set to about 0.5 or more, about 0.6 or more, about 0.7 or more, about 0.8 or more, or about 0.9 or more, and more preferably set to about 0.6 or more.

[Step (b)]

The method for selecting a biomarker for predicting a content of IL-6 comprises clustering the subject group based on the content of the biomarker candidate substance selected in (a).

In one embodiment, clustering is preferably a classification by unsupervised machine learning for the data of the content of all the biomarker candidate substances selected in (a).

In one embodiment of this case, the clustering may be, for example, a clustering by a hierarchical clustering method or a non-hierarchical clustering method such as k-means method or k-medoids method, and is not limited to the extent that the desired clustering is possible. It is preferred, however, that the clustering is a clustering by a non-hierarchical clustering method, more preferably a clustering by k-medoids method.

The number of clusters of a subject group can be determined by those skilled in the art according to a conventional method in the art, and may be clustered into two clusters, three clusters, or four clusters, preferably clustered into three clusters.

In one embodiment, when clustering is performed, for example, using the k-means method or the k-medoids method, the number of clusters needs to be set at the time of performing.

[Step (c)]

In one embodiment, it is preferred that the method for selecting a biomarker for predicting a content of IL-6 further comprises [c] selecting a biomarker candidate substance selected in (a) as a biomarker candidate substance when the biomarker candidate substance selected in (a) is capable of discriminating a subject with high reliability in comparison with a result of clustering in (b).

In one embodiment, the method for selecting a biomarker for predicting a content of IL-6 may be the selecting method comprising, in (c),

(d) comparing a result of discriminating the subject using each of a series of any threshold candidate values of the biomarker candidate substance selected in (a) with the result of clustering in (b), thereby acquiring a sensitivity and a specificity of the discrimination using each threshold candidate value of the biomarker candidate substance;
(e) acquiring a reliability based on the sensitivity and the specificity acquired in (d); and
(f) determining whether or not the biomarker candidate substance is capable of discriminating the subject with high reliability.

In one embodiment, it is preferred that the reliability is indicated by AUC of a ROC curve acquired based on a sensitivity and a specificity.

In one embodiment, in (f), it is determined that the biomarker candidate is capable of discriminating the subject with high reliability, preferably when the AUC is about 0.7 or more, about 0.8 or more, or about 0.9 or more, and more preferably when the AUC is about 0.8 or more.

In one embodiment, the method for selecting a biomarker for predicting a content of IL-6 may be a computer-implemented method implemented by a computer.

Herein, an embodiment of (c) comprising (d) to (f), will be described, for example, assuming that the subject group is clustered into three clusters based on the content of the biomarker candidate substance in (b).

Of the three clusters obtained by clustering, the cluster having a high content of the biomarker candidate substance (including one or two clusters) can be set as the cluster of the "subject having a high content of IL-6", and the other cluster can be set as the cluster of the "subject having a low content of IL-6", which is a "result of clustering in (b)".

In (d), the "sensitivity" and "specificity" of the "biomarker candidate substance selected in (a)" can be acquired, for example, as follows.

First, for the content of a biomarker candidate substance selected in (a), a series of any threshold candidate values is set, and each subject is discriminated whether the subject is a "subject having a high content of IL-6" whose content is greater than or equal to the threshold candidate value (or greater than the threshold candidate value), or a "subject having a low content of IL-6" whose content is less than the threshold candidate value (or less than or equal to the threshold candidate value). Then, by comparing the result of discriminating the "subject having a high content of IL-6" and the "subject having a low content of IL-6 using each threshold candidate value with the results of clustering the "subject having a high content of IL-6 and the "subject having a low content of IL-6" (the result of clustering in (b)), the sensitivity and the specificity of the discrimination of "subject having a high content of IL-6 and "subject having a low content of IL-6 can be acquired in the case where each threshold candidate value is used.

More specifically, by comparing the result of discriminating the "subject having a high content of IL-6" by a certain threshold candidate value with the result of clustering the "subject having a high content of IL-6" (the result of clustering in (b)), the probability (sensitivity) that the "subject having a high content of IL-6 can be correctly discriminated by the threshold candidate value can be obtained. Furthermore, by comparing the result of discriminating the "subject having a low content of IL-6" by a certain threshold candidate value with the result of clustering the "subject having a low content of IL-6" (the result of clustering in (b)), the probability (specificity) that the "subject having a low content of IL-6 can be correctly discriminated by the threshold candidate value can be obtained.

This method is repeated to obtain a sensitivity and a specificity corresponding to each of the series of any threshold candidate values.

In (e), a reliability is acquired based on the sensitivity and the specificity acquired in (d). The reliability can be indicated, for example, by the area under the curve (AUC) of the ROC curve drawn based on the sensitivity and the specificity.

In other words, the AUC thus acquired shows the reliability of the result of discriminating using the biomarker candidate substance to discriminate the subject compared with the result of clustering at (b).

In (f), whether or not the biomarker candidate substance is capable of discriminating a subject with high reliability is determined. This determination is preferably based on the reliability indicated by the AUC as described above. Then, when the AUC is about 0.7 or more, about 0.8 or more, or about 0.9 or more, more preferably when the AUC is about 0.8 or more, it is determined that the biomarker candidate is capable of discriminating the subject with high reliability.

With the above-described method for selecting a biomarker for predicting a content of IL-6, it is possible to select a biomarker that is effective for predicting a content of IL-6 in a sample acquired from a subject.

= = Device for selecting biomarker for predicting content of IL-6 = =

The device for selecting a biomarker for predicting a content of IL-6 according to the present embodiment is a device for carrying out the method described in the section "Method for selecting biomarker for predicting content of IL-6".

That is, the device for selecting a biomarker for predicting a content of IL-6 according to the present embodiment is
a selecting device comprising:

　　(a) a selection unit that selects a biomarker candidate substance based on a vector similarity acquired using a content of IL-6 and a content of the biomarker candidate substance in a sample collected from a subject group; and
　　(b) a clustering unit that clusters the subject group based on the content of the biomarker candidate substance selected in (a).

In one embodiment, the device for selecting a biomarker for predicting a content of IL-6 is
the selecting device further comprising (c) a selection unit that selects a biomarker candidate substance selected in (a) as a biomarker candidate substance when the biomarker candidate substance selected in (a) is capable of discriminating a subject with high reliability in comparison with a result of clustering in (b).

In one embodiment, the device for selecting a biomarker for predicting a content of IL-6 is
the selecting device, wherein the selection unit (c) comprises:

　　(d) an acquisition unit that compares a result of discriminating the subject using each of a series of any threshold candidate values of the biomarker candidate substance selected in (a) with the result of clustering in (b), thereby acquiring a sensitivity and a specificity of the discrimination using each threshold candidate value of the biomarker candidate substance;
　　(e) an acquisition unit that acquires a reliability based on the sensitivity and the specificity acquired in (d); and
　　(f) a determination unit that determines whether or not the biomarker candidate substance is capable of discriminating the subject with high reliability.

In the device for selecting a biomarker for predicting a content of IL-6, each embodiment and a preferred embodiment of "subject group", "IL-6-related disease" and the like and in the steps (a) to (f) are as described in the section "Method for selecting biomarker for predicting content of IL-6".

= = Method for selecting biomarker for predicting responsiveness of subject to treatment with IL-6 signaling pathway inhibitor = =

In one embodiment, the "method for selecting a biomarker for predicting a content of IL-6" can be used as a method for selecting a biomarker for predicting a responsiveness of a subject to a treatment with an IL-6 signaling pathway inhibitor, the method being capable of predicting IL-6 as a biomarker.

That is, the method for selecting a biomarker for predicting a responsiveness of a subject to a treatment with an IL-6 signaling pathway inhibitor is a selecting method comprising:

　　(a) selecting a biomarker candidate substance based on a vector similarity acquired using a content of IL-6 and a content of a biomarker candidate substance in a sample collected from a subject group; and
　　(b) clustering the subject group based on the content of the biomarker candidate substance selected in (a).

In one embodiment, the method for selecting a biomarker for predicting a responsiveness of a subject to a treatment with an IL-6 signaling pathway inhibitor is a selecting method further comprising (c) selecting a biomarker candidate substance selected in (a) as a biomarker candidate substance when the biomarker candidate substance selected in (a) is capable of discriminating a subject with high reliability in comparison with a result of clustering in (b).

In one embodiment, the method for selecting a biomarker for predicting a responsiveness of a subject to a treatment with an IL-6 signaling pathway inhibitor is the selecting method comprising, in (c),

(d) comparing a result of discriminating the subject using each of a series of any threshold candidate values of the biomarker candidate substance selected in (a) with the result of clustering in (b), thereby acquiring a sensitivity and a specificity of the discrimination using each threshold candidate value of the biomarker candidate substance;
(e) acquiring a reliability based on the sensitivity and the specificity acquired in (d); and
(f) determining whether or not the biomarker candidate substance is capable of discriminating the subject with high reliability.

Thus, except the matters specified below, in the method for selecting a biomarker for predicting a responsiveness of a subject to a treatment with an IL-6 signaling pathway inhibitor, each embodiment and a preferred embodiment of "subject group", "IL-6-related disease" and the like and in the steps (a) to (f) are as described in the section "Method for selecting biomarker for predicting content of IL-6".

Further, the "IL-6 signaling pathway inhibitor" is as described in the section "Explanation of terms".

In one embodiment, the subject group in the method for selecting a biomarker for predicting a responsiveness of a subject to a treatment with an IL-6 signaling pathway inhibitor is preferably a subject group suspected of suffering from an IL-6-related disease or a subject group suffering from an IL-6-related disease.

Accordingly, in one embodiment, when the subject group is a subject group suspected of suffering from an IL-6-related disease, the method for selecting a biomarker for predicting a responsiveness of a subject to a treatment with an IL-6 signaling pathway inhibitor can be used as a method for selecting a biomarker for predicting whether or not each subject constituting the subject group suspected of suffering from an IL-6-related disease suffers from the IL-6-related disease.

In this case, the IL-6-related disease is preferably a disease in which the IL-6 content or the presence or absence of IL-6 in a sample changes by whether or not the subject suffers from the disease.

In another embodiment, when the subject group is a subject group suffering from an IL-6-related disease, the method for selecting a biomarker for predicting a responsiveness of a subject to a treatment with an IL-6 signaling pathway inhibitor can be used as a method for selecting a biomarker for predicting whether or not each subject constituting the subject group suffering from an IL-6-related disease is an individual having a responsiveness to a treatment especially with an IL-6 signaling pathway substance.

In this case, the IL-6-related disease is preferably a disease in which the IL-6 content or the presence or absence of IL-6 in a sample changes based on the responsiveness to a treatment with an IL-6 signaling pathway substance of each subject in the subject group suffering from an IL-6-related disease.

In one embodiment, the subject group in the method for selecting a biomarker for predicting a responsiveness of a subject to a treatment with an IL-6 signaling pathway inhibitor is preferably a subject group suffering from PAH.

It has been reported that IL-6 in blood is elevated and expression of IL-6 receptors in diseased organs is high in PAH, and that treatments with IL-6 signaling pathway inhibitors are effective in PAH. That is, it is recognized that PAH can be treated by inhibition of IL-6 action, and the content of IL-6 in a subject suffering from PAH would be an indicator of responsiveness to a treatment with an IL-6 signaling pathway inhibitor.

Thus, the method for selecting a biomarker according to the present embodiment can be used for the purpose of acquiring a biomarker for predicting a responsiveness of a subject suffering from PAH to a treatment with an IL-6 signaling pathway inhibitor and/or for the purpose of acquiring a biomarker that can indicate a responsiveness of a subject to a treatment with an IL-6 signaling pathway inhibitor in combination with IL-6 or in place of IL-6.

= = Device for selecting biomarker for predicting responsiveness of subject to treatment with IL-6 signaling pathway inhibitor = =

The device for selecting a biomarker for predicting a responsiveness to a treatment with an IL-6 signaling pathway inhibitor according to the present embodiment is a device for carrying out the method described in the section "Method for selecting biomarker for predicting responsiveness to treatment with IL-6 signaling pathway inhibitor".

Then, the "Device for selecting biomarker for predicting content of IL-6 " can be used as a device for selecting a biomarker for predicting a responsiveness to a treatment with an IL-6 signaling pathway inhibitor.

That is, the device for selecting a biomarker for predicting a responsiveness to a treatment with an IL-6 signaling pathway inhibitor according to the present embodiment is a selecting device comprising:

(a) a selection unit that selects a biomarker candidate substance based on a vector similarity acquired using a content of IL-6 and a content of the biomarker candidate substance in a sample collected from a subject group; and
(b) a clustering unit that clusters the subject group based on the content of the biomarker candidate substance selected in (a).

In one embodiment, the device for selecting a biomarker for predicting a responsiveness to a treatment with an IL-6 signaling pathway inhibitor is
the selecting device further comprising (c) a selection unit that selects a biomarker candidate substance selected in (a) as a biomarker candidate substance when the biomarker candidate substance selected in (a) is capable of discriminating a subject with high reliability in comparison with a result of clustering in (b).

In one embodiment, the device for selecting a biomarker for predicting a responsiveness to a treatment with an IL-6 signaling pathway inhibitor is
the selecting device, wherein the selection unit (c) comprises:

(d) an acquisition unit that compares a result of discriminating the subject using each of a series of any threshold candidate values of the biomarker candidate substance selected in (a) with the result of clustering in (b), thereby acquiring a sensitivity and a specificity of the discrimination using each threshold candidate value of the biomarker candidate substance;
(e) an acquisition unit that acquires a reliability based on the sensitivity and the specificity acquired in (d); and
(f) a determination unit that determines whether or not the biomarker candidate substance is capable of discriminating the subject with high reliability.

In the device for selecting a biomarker for predicting a responsiveness to a treatment with an IL-6 signaling pathway inhibitor, each embodiment and a preferred embodiment of "subject group", "IL-6-related disease" and the like and in the steps (a) to (f) are as described in the section "Method for selecting biomarker for predicting responsiveness to treatment with IL-6 signaling pathway inhibitor".

= = Method for selecting biomarker for selecting subject who is likely to have responsiveness to treatment with IL-6 signaling pathway inhibitor = =

By predicting a responsiveness of a subject to a treatment with an IL-6 signaling pathway inhibitor, a subject who is likely to have a responsiveness to a treatment with an IL-6 signaling pathway inhibitor can be selected.

Thus, in one embodiment, the "Method for selecting biomarker for predicting responsiveness of subject to treatment with IL-6 signaling pathway inhibitor" can be used as a method for selecting a biomarker for selecting a subject who is likely to have a responsiveness to a treatment with an L-6 signaling pathway inhibitor.

That is the method for selecting a biomarker for selecting a subject who is likely to have a responsiveness to a treatment with an L-6 signaling pathway inhibitor is a selecting method comprising:

(a) selecting a biomarker candidate substance based on a vector similarity acquired using a content of IL-6 and a content of a biomarker candidate substance in a sample collected from a subject group; and
(b) clustering the subject group based on the content of the biomarker candidate substance selected in (a).

In one embodiment, the method for selecting a biomarker for selecting a subject who is likely to have a responsiveness to a treatment with an L-6 signaling pathway inhibitor is the selecting method further comprising (c) selecting a biomarker candidate substance selected in (a) as a biomarker candidate substance when the biomarker candidate substance selected in (a) is capable of discriminating a subject with high reliability in comparison with a result of clustering in (b).

In one embodiment, the method for selecting a biomarker for selecting a subject who is likely to have a responsiveness to a treatment with an L-6 signaling pathway inhibitor is the selecting method comprising, in (c),

(d) comparing a result of discriminating the subject using each of a series of any threshold candidate values of the biomarker candidate substance selected in (a) with the result of clustering in (b), thereby acquiring a sensitivity and a specificity of the discrimination using each threshold candidate value of the biomarker candidate substance;
(e) acquiring a reliability based on the sensitivity and the specificity acquired in (d); and
(f) determining whether or not the biomarker candidate substance is capable of discriminating the subject with high reliability.

Thus, except the matters specified below, in the method for selecting a biomarker for selecting a subject who is likely to have a responsiveness to a treatment with an L-6 signaling pathway inhibitor, each embodiment and a preferred embodiment of "subject group", "IL-6-related disease" and the like and in the steps (a) to (f) are as described in the section "Method for selecting biomarker for predicting responsiveness of subject to treatment with IL-6 signaling pathway inhibitor".

= = Device for selecting biomarker for selecting subject who is likely to have responsiveness to treatment with IL-6 signaling pathway inhibitor = =

The device for selecting a biomarker for selecting a subject who is likely to have a responsiveness to a treatment with an IL-6 signaling pathway inhibitor according to the present embodiment is a device for carrying out the method described in the "Method for selecting biomarker for selecting subject who is likely to have responsiveness to treatment with IL-6 signaling pathway inhibitor".

By predicting a responsiveness of a subject to a treatment with an IL-6 signaling pathway inhibitor, a subject who is likely to have a responsiveness to a treatment with an IL-6 signaling pathway inhibitor can be selected.

Thus, in one embodiment, the "Device for selecting biomarker for predicting responsiveness of subject to treatment with IL-6 signaling pathway inhibitor" can be used as a device for selecting a biomarker for selecting a subject who is likely to have a responsiveness to a treatment with an IL-6 signaling pathway inhibitor.

That is, the device for selecting a biomarker for selecting a subject who is likely to have a responsiveness to a treatment with an IL-6 signaling pathway inhibitor according to the present embodiment is a selecting device comprising:

(a) a selection unit that selects a biomarker candidate substance based on a vector similarity acquired using a content of IL-6 and a content of the biomarker candidate substance in a sample collected from a subject group; and
(b) a clustering unit that clusters the subject group based on the content of the biomarker candidate substance selected in (a).

In one embodiment, the device for selecting a biomarker for selecting a subject who is likely to have a responsiveness to a treatment with an IL-6 signaling pathway inhibitor is the selecting device further comprising (c) a selection unit that selects a biomarker candidate substance selected in (a) as a biomarker candidate substance when the biomarker candidate substance selected in (a) is capable of discriminating a subject with high reliability in comparison with a result of clustering in (b).

In one embodiment, the device for selecting a biomarker for selecting a subject who is likely to have a responsiveness to a treatment with an IL-6 signaling pathway inhibitor is the selecting device, wherein the selection unit (c) comprises:

(d) an acquisition unit that compares a result of discriminating the subject using each of a series of any threshold candidate values of the biomarker candidate substance selected in (a) with the result of clustering in (b), thereby acquiring a sensitivity and a specificity of the discrimination using each threshold candidate value of the biomarker candidate substance;
(e) an acquisition unit that acquires a reliability based on the sensitivity and the specificity acquired in (d); and
(f) a determination unit that determines whether or not the biomarker candidate substance is capable of discriminating the subject with high reliability.

In the device for selecting a biomarker for selecting a subject who is likely to have a responsiveness to a treatment with an IL-6 signaling pathway inhibitor, each embodiment and a preferred embodiment of "subject group", "IL-6-related disease" and the like and in the steps (a) to (f) are as described in the section "Device for selecting biomarker for predicting responsiveness of subject to treatment with IL-6 signaling pathway inhibitor" or "Method for selecting biomarker for predicting responsiveness of subject to treatment with IL-6 signaling pathway inhibitor".

= = Biomarker for predicting content of IL-6 in subject = =

The biomarker for predicting a content of IL-6 in a subject according to the present embodiment comprises one or more cytokines selected from the group consisting of CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1.

Use of a biomarker according to another embodiment is use of a biomarker comprising one or more cytokines selected from the group consisting of CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1, for predicting a content of IL-6 in a subject.

Regarding the above "biomarker" and "use", in one embodiment, the biomarker may comprise one or more cytokines or a combination of two or more cytokines selected based on any conditions such as reliability of prediction, application, economy, and/or simplicity of measurement.

For example, when the ease of measurement and/or economy is particularly considered, the biomarker for predicting a content of IL-6 in a subject preferably comprises IL-1β/IL-1F2 or IL-4, and may comprise a combination of IL-1β/IL-1F2 and IL-4.

[Subject]

In the biomarker for predicting a content of IL-6 in a subject, the "subject" is not limited as long as it is an organism in which IL-6 and at least one of the biomarkers other than IL-6 is present in vivo on a constant or temporary basis. For example, the subject may be a mammal or an animal other than a mammal. The mammal may be a human or a non-human animal, and the non-human animal species may be, for example, a monkey, a dog, a cat, a horse, a cow, a pig, a sheep, a goat, a rabbit, a guinea pig, a hamster, a mouse, and/or a rat. The subject is not limited by its use such as a domestic animal, a pet animal, or a laboratory animal, but is preferably a mammal, and more preferably a human.

That is, in one embodiment, the cytokine for predicting a content of IL-6 in a subject is preferably a cytokine derived from a human.

The content of IL-6 is preferably a content of IL-6 in blood (whole blood), plasma, of serum of a subject, more preferably a content of IL-6 in blood (whole blood), plasma, or serum of a human subject.

Further, the cytokine for predicting a content of IL-6 is preferably a cytokine contained in blood (whole blood), plasma, or serum of a subject, more preferably a cytokine contained in blood (whole blood), plasma, or serum of a human subject.

The subject may be a subject extracted based on the state of the subject or a randomly selected subject, and those skilled in the art may appropriately determine the subject to the extent that at least one of the biomarkers may become an indicator for predicting the content of IL-6.

Here, the state can be any state of the subject, for example, may be a state related to a particular disease or an unrelated state.

When the state is a state related to a particular disease, the state is not limited as long as the state is at least partially related to the particular disease. Examples of the state related to a particular disease include a state in which a subject is suspected of suffering from a particular disease, a state in which whether or not a subject is suffering from a particular disease, a state in which whether or not a subject is suffering from any subtype of a particular disease, a state in which whether or not a subject suffering from a particular disease is responsive to a certain treatment, a state of progression of the particular disease which the subject is suffering from, and/or a state of improvement of a symptom of a particular disease which the subject is suffering from.

When the state of the subject is a state unrelated to a particular disease, examples of the state of the subject include physiological characteristics, genetic conditions such as sex or race, age, and/or geographical conditions such as place of residence and area of activity, which are unrelated to any disease.

[IL-6-related disease]

In one embodiment, the subject is preferably a subject suspected of suffering from an IL-6-related disease or a subject suffering from an IL-6-related disease. Here, the "IL-6-related disease" is as described in the section "Explanation of terms".

In one embodiment, an IL-6-related disease may be a disease in which the IL-6 content or the presence or absence of IL-6 in a sample changes by whether or not the subject is related to the disease, or a disease in which the content of IL-6 or the presence or absence of IL-6 in a sample changes based on the state of the subject in a group of subjects suffering from the IL-6-related disease, and the state of the subject can be classified into subtypes based on such changes.

In one embodiment, it is preferred that the IL-6-related disease is pulmonary hypertension. In other words, the subject is preferably a subject suffering from pulmonary hypertension.

Here, pulmonary hypertension is clinically classified into pulmonary arterial hypertension (PAH, group 1), pulmonary hypertension with left cardiac heart disease (group 2), pulmonary hypertension with pulmonary disease and/or hypoxemia (group 3), CTEPH (group 4), and pulmonary hypertension with an unspecified multifactorial mechanism (group 5) (Guidelines for Treatment of Pulmonary Hypertension (revised version in 2017)).

In one embodiment, the pulmonary hypertension is more preferably pulmonary arterial hypertension (PAH). In other words, the subject is preferably a subject suffering from PAH.

The present inventors have found that CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1 exhibit similar behavior to IL-6 in a patient suffering from PAH, and would be an indicator of a responsiveness to a treatment with an IL-6 signaling pathway substance.

Thus, in a patient suffering from PAH, the content of IL-6 can be predicted by one or more cytokines selected from the group consisting of CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1.

It has been reported that IL-6 in blood is elevated and expression of IL-6 receptors in diseased organs is high in PAH, and that treatments with IL-6 signaling pathway inhibitors are effective in PAH. That is, it is recognized that PAH can be treated by inhibition of IL-6 action, and the content of IL-6 in a subject suffering from PAH would be an indicator of responsiveness to a treatment with an IL-6 signaling pathway inhibitor.

Thus, in one embodiment, when the subject is a subject suffering from PAH, the biomarker according to the present embodiment can be used for the purpose of predicting a content of IL-6 and/or for the purpose of predicting a responsiveness of a subject to a treatment with an IL-6 signaling pathway inhibitor in combination with IL-6 or in place of IL-6.

= = Method for predicting content of IL-6 in subject = =

The biomarker for predicting a content of IL-6 in a subject can be used for predicting the content of IL-6 in the subject.

Accordingly, the method for predicting a content of IL-6 in a subject according to the present embodiment is a method comprising measuring a content of the biomarker described in the "Biomarker for predicting content of IL-6 in subject" in a sample collected from a subject.

In one embodiment, the method for predicting a content of IL-6 in a subject preferably comprises predicting the content of IL-6 based on the content of the biomarker.

Each embodiment and a preferred embodiment of the "measurement" in the present embodiment are as described in the section "Method for measuring biomarker".

Further, each embodiment and a preferred embodiment of the "subject", "IL-6-related disease" and the like are as described in the section "Biomarker for predicting content of IL-6 in subject".

Here, more specifically, regarding at least one biomarker described in the section "Biomarker for predicting content of IL-6 in subject", which would be an indicator of the behavior of IL-6 in the subject, a corresponding data may be acquired by measuring the content of IL-6 and the content of the biomarker in advance.

This allows, regarding the subject to be tested, measuring a content of a biomarker and predicting the corresponding content of IL-6.

Reference value (threshold) of the content of the biomarker, which would be a reference value for predicting the content of IL-6, can be set appropriately by those skilled in the art, depending on the degree of the sensitivity and/or the specificity at which the responsiveness is desired to be predicted.

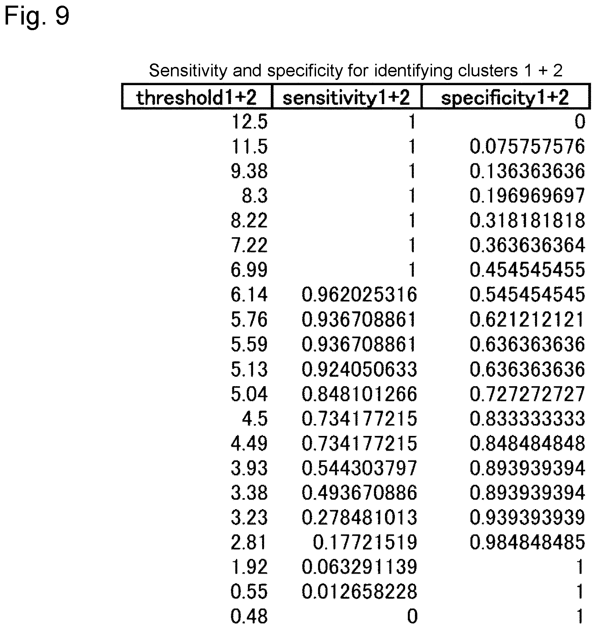

For example, when the subject is a subject suffering from PAH, in other words, the method according to the present embodiment is a method for predicting the content of IL-6 in a subject suffering from PAH, the threshold of the blood content of the biomarker, which is IL-1β, can be suitably selected by those skilled in the art with reference to the threshold, sensitivity and specificity as shown in Figure 9, considering how or to what extent the subject group is to be discriminated by IL-1β. For example, the sensitivity and the specificity may be selected so as to maximize the sensitivity and/or the specificity, or maximize the sum of the sensitivity and the specificity.

Specifically, in one embodiment, for the blood content of IL-1β, the threshold is preferably a threshold corresponding to the case where both a sensitivity and a specificity are high, for example, preferably a threshold corresponding to the case where the sum of the sensitivity and the specificity is about 1.3 to about 1.6, and more preferably a threshold corresponding to the case where the sum of the sensitivity and the specificity is about 1.4 to about 1.6. The sensitivity and the specificity may then be selected so that either the sensitivity or the specificity is relatively high, considering how or to what extent the subject group is to be discriminated by the IL-1β.

More specifically, for the serum content of IL-1β, the threshold may be about 3.0 to about 9.0 (pg/mL), about 3.5 to about 7.0 (pg/mL), or about 4.0 to about 6.0 (pg/mL). In one embodiment, when referring to Figure 9, the threshold of serum content of IL-1β may be selected as 4.49 (pg/mL), 4.50 (pg/mL), 5.04 (pg/mL), 5.13 (pg/mL), 5.59 (pg/mL), or 5.76 (pg/mL).

= = Biomarker for predicting responsiveness of subject to treatment with IL-6 signaling pathway inhibitor = =

In one embodiment, the "Biomarker for predicting content of IL-6 in subject" and IL-6 can be used as biomarkers for predicting a responsiveness of a subject to a treatment with an IL-6 signaling pathway inhibitor.

That is, the biomarker for predicting a responsiveness of a subject to a treatment with an IL-6 signaling pathway inhibitor according to the present embodiment comprises one or more cytokines selected from the group consisting of IL-6, CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1.

The use of biomarker according to another embodiment is use of a biomarker comprising one or more cytokines selected from the group consisting of IL-6, CXCL9/MIG, CCL4/MIP- 10, CCL27/CTACK, IL-1β/IL-1F2, GDF-15, IL-4, G-CSF, CXCL10/1P-10/CRG-2, and IL-1α/IL-1F1, for predicting a responsiveness of a subject to a treatment with an IL-6 signaling pathway inhibitor.

Regarding the above "biomarker" and "use", in one embodiment, the biomarker for predicting a responsiveness of a subject to a treatment with an IL-6 signaling pathway inhibitor according to the present embodiment may comprise one or more cytokines or a combination of two or more cytokines selected based on any conditions such as reliability of prediction, application, economy, and/or simplicity of measurement.

For example, when the ease of measurement and/or economy is particularly considered, the biomarker for predicting a responsiveness of a subject to a treatment with an IL-6 signaling pathway inhibitor preferably comprises IL-6, IL-1β/IL-1F2, or IL-4, and may comprise a combination of two or three cytokines of these. That is, the biomarker may comprise a combination of IL-6 and IL-1β/IL-1F2, a combination of IL-6 and IL-4, a combination of IL-1 β/IL-1F2 and IL-4, or a combination of IL-6, IL-1β/IL-1F2, and IL-4.

In the present invention, except the matters specified below, each embodiment and a preferred embodiment of the "subject", "IL-6-related disease" and the like are as described in the section "Biomarker for predicting content of IL-6 in subject".

Further, the "IL-6 signaling pathway inhibitor" is as described in the section "Explanation of terms" above.

Since the biomarker according to the present embodiment is a biomarker for predicting a responsiveness of a subject to a treatment with an IL-6 signaling pathway inhibitor, the subject is in a state in which the treatment with an IL-6 signaling pathway inhibitor can be applied.

In one embodiment, such a state includes, for example, a state in which the subject is suspected of suffering from a disease to which the treatment with an IL-6 signaling pathway inhibitor may be applied, or a state in which the subject is suffering from a disease to which the treatment with an IL-6 signaling pathway inhibitor may be applied.

In one embodiment, the disease is PAH. Accordingly, the biomarker according to the present embodiment can be suitably used as a biomarker for predicting a responsiveness of a subject suffering from PAH to a treatment with an IL-6 signaling pathway inhibitor.

It has been reported that IL-6 in blood is elevated and expression of IL-6 receptors in diseased organs is high in PAH, and that treatments with IL-6 signaling pathway inhibitors are effective in PAH. That is, it is recognized that PAH can be treated by inhibition of IL-6 action, and the content of IL-6 in a subject suffering from PAH would be an indicator of responsiveness to a treatment with an IL-6 signaling pathway inhibitor.

The present inventors have further found that CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1 exhibit similar behavior to IL-6 in a patient suffering from PAH, and would be an indicator of a responsiveness to a treatment with an IL-6 signaling pathway substance.

Thus, in a subject suffering from PAH, a responsiveness to a treatment with an IL-6 signaling pathway inhibitor can be predicted by the biomarker comprising one or more cytokines selected from the group consisting of IL-6, CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1.

= = Method for predicting responsiveness of subject to treatment with IL-6 signaling pathway inhibitor = =

By predicting a responsiveness of a subject to a treatment with an IL-6 signaling pathway inhibitor, a subject who is likely to have a responsiveness to a treatment with an IL-6 signaling pathway inhibitor can be selected.

Accordingly, the method for predicting a responsiveness of a subject to a treatment with an IL-6 signaling pathway inhibitor according to the present embodiment is a method comprising measuring a content of a biomarker described in the section "Biomarker for predicting responsiveness of subject to treatment with IL-6 signaling pathway inhibitor" in a sample collected from a subject.

In one embodiment, the method for predicting a responsiveness of a subject to a treatment with an IL-6 signaling pathway inhibitor preferably comprises determining whether or not the subject is likely to have a responsiveness to a treatment with an IL-6 signaling pathway inhibitor, based on the content of the biomarker.

Each embodiment and a preferred embodiment of the "measurement" in the present embodiment are as described in the section "Method for measuring biomarker".

Each embodiment and a preferred embodiment of the "subject", "IL-6-related disease", "IL-6 signaling pathway inhibitor" and the like in the present embodiment are as described in the section "Biomarker for predicting responsiveness of subject to treatment with IL-6 signaling pathway inhibitor".

Here, more specifically, regarding at least one biomarker described in the section "Biomarker for predicting responsiveness of subject to treatment with IL-6 signaling pathway inhibitor", which would be an indicator of a responsiveness of the subject to a treatment with IL-6 signaling pathway inhibitor, a corresponding data that makes correspondence between the responsiveness to an IL-6 signaling pathway inhibitor and the content of the biomarker may be acquired in advance.

This allows, regarding the subject to be tested, measuring a content of a biomarker and predicting the corresponding responsiveness thereto.

Reference value (threshold) of the content of the biomarker, which would be a reference value for the presence or absence of responsiveness, can be set appropriately by those skilled in the art, depending on the degree of the sensitivity and/or the specificity at which the responsiveness is desired to be predicted.

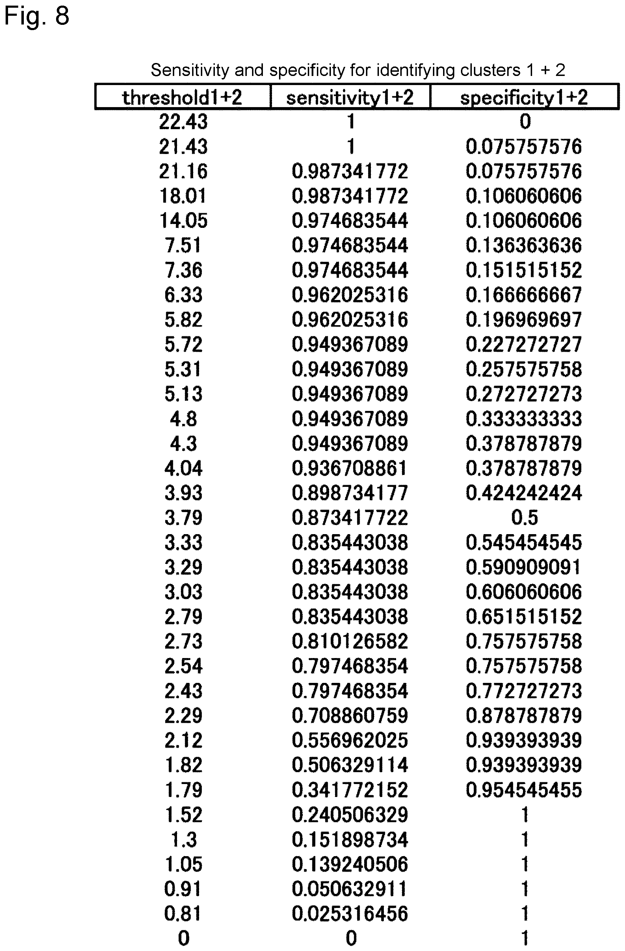

For example, when the subject is a subject suffering from PAII, in other words, the method according to the present embodiment is a method for predicting a responsiveness of a subject suffering from PAH to a treatment with an IL-6 signaling pathway inhibitor, the threshold of the blood content of the biomarker, which is IL-6, can be suitably selected by those skilled in the art with reference to the threshold, sensitivity and specificity as shown in Figure 8, considering how or to what extent the subject group is to be discriminated by IL-6. For example, the sensitivity and the specificity may be selected so as to maximize the sensitivity and/or the specificity, or maximize the sum of the sensitivity and the specificity.

Specifically, in one embodiment, for the blood content of IL-6, the threshold is preferably a threshold corresponding to the case where both the sensitivity and the specificity are relatively high, for example, preferably a threshold corresponding to the case where the sum of the sensitivity and the specificity is about 1.3 to about 1.6, and more preferably a threshold corresponding to the case where the sum of the sensitivity and the specificity is about 1.4 to about 1.6. The sensitivity and the specificity may then be selected so that either the sensitivity or the specificity is relatively high, considering how or to what extent the subject group is to be discriminated by IL-6.

More specifically, for the serum content of IL-6, the threshold may be about 1.0 to about 5.0 (pg/mL), about 1.5 to about 4.0 (pg/mL), or about 2.0 to about 3.0 (pg/mL). In one embodiment, when referring to Figure 8, the threshold of serum content of IL-6 may be selected as 2.12 (pg/mL), 2.29 (pg/mL), 2.43 (pg/mL), 2.54 (pg/mL), 2.73 (pg/mL), or 2.79 (pg/mL).

Further, for example, when the subject is a subject suffering from PAH, in other words, the method according to the present embodiment is a method for predicting a responsiveness of a subject suffering from PAH to a treatment with an IL-6 signaling pathway inhibitor, the threshold of the blood content of the biomarker, which is IL-1β, can be suitably selected by those skilled in the art with reference to the threshold, sensitivity and specificity as shown in Figure 9, considering how or to what extent the subject group is to be discriminated by IL-1β.

For example, the sensitivity and the specificity may be selected so as to maximize the sensitivity and/or the specificity, or maximize the sum of the sensitivity and the specificity.

Specifically, in one embodiment, for the blood content of IL-1β, the threshold is preferably a threshold corresponding to the case where both the sensitivity and the specificity are high, for example, preferably a threshold corresponding to the case where the sum of the sensitivity and the specificity is about 1.3 to about 1.6, and more preferably a threshold corresponding to the case where the sum of the sensitivity and the specificity is about 1.4 to about 1.6. The sensitivity and the specificity may then be selected so that either the sensitivity or the specificity is relatively high, considering how or to what extent the subject group is to be discriminated by the IL-1β.

More specifically, for the serum content of IL-1β, the threshold may be about 3.0 to about 9.0 (pg/mL), about 3.5 to about 7.0 (pg/mL), or about 4.0 to about 6.0 (pg/mL). In one embodiment, when referring to Figure 9, the threshold of content of IL-1β may be selected as 4.49 (pg/mL), 4.50 (pg/mL), 5.04 (pg/mL), 5.13 (pg/mL), 5.59 (pg/mL), or 5.76 (pg/mL).

= = Biomarker for selecting subject who is likely to have responsiveness to treatment with IL-6 signaling pathway inhibitor = =

By predicting a responsiveness of a subject to a treatment with an IL-6 signaling pathway inhibitor, a subject who is likely to have a responsiveness to a treatment with an IL-6 signaling pathway inhibitor can be selected.

Thus, in one embodiment, the "biomarker for predicting a responsiveness of a subject to a treatment with an IL-6 signaling pathway inhibitor" can be used as a biomarker for selecting a subject who is likely to have a responsiveness to a treatment with an IL-6 signaling pathway inhibitor.

In the biomarker for selecting a subject who is likely to have a responsiveness to a treatment with an IL-6 signaling pathway inhibitor, each embodiment and a preferred embodiment of the "subject", "IL-6-related disease", "IL-6 signaling pathway inhibitor" and the like are as described in the section "Biomarker for predicting responsiveness of subject to treatment with IL-6 signaling pathway inhibitor".

As described above, in a patient suffering from PAH, a responsiveness to a treatment with an IL-6 signaling pathway inhibitor can be predicted by one or more cytokines selected from the group consisting of IL-6, CXCL9/MIG, CCL4/MIP-1β, CCL27/CTACK, IL-1 β/IL-1F2, GDF-15, IL-4, G-CSF, CXCL10/1P-10/CRG-2, and IL-1α/IL-1F1, or by a combination of two or more cytokines of these, or by any of the combinations exemplified above. For example, when the ease of measurement and/or economy is particularly considered, the combination preferably comprises IL-6, IL-1β/IL-1F2, or IL-4, and may comprise a combination of two or three cytokines of these. That is, the combination may be a combination of IL-6 and IL-1β/IL-1F2, a combination of IL-6 and IL-4, a combination of IL-1β/IL-1F2 and IL-4, or a combination of IL-6, IL-1β/IL-1F2, and IL-4.

Then, based on the prediction, a subject who is likely to have a responsiveness to a treatment with IL-6 signaling pathway inhibitor can be selected.

= = Method for selecting subject who is likely to have responsiveness to treatment with IL-6 signaling pathway inhibitor = =

The "biomarker for selecting a subject who is likely to have a responsiveness of a subject to a treatment with an IL-6 signaling pathway inhibitor" can be used for selecting a subject who is likely to have a responsiveness to a treatment with an IL-6 signaling pathway inhibitor.

Accordingly, the method for selecting a subject who is likely to have a responsiveness to a treatment with an IL-6 signaling pathway inhibitor according to the present embodiment is a method comprising measuring a content of a biomarker described in the section "Biomarker for selecting subject who is likely to have responsiveness to treatment with IL-6 signaling pathway inhibitor" in a sample collected from a subject.

In one embodiment, the method for selecting a subject who is likely to have a responsiveness to a treatment with an IL-6 signaling pathway inhibitor preferably further comprises determining whether or not the subject is likely to have a responsiveness to a treatment with an IL-6 signaling pathway inhibitor, based on the content of the biomarker.

In one embodiment, it is more preferred that the method for selecting a subject who is likely to have a responsiveness to a treatment with an IL-6 signaling pathway inhibitor further comprises selecting a subject who is determined as likely to have a responsiveness to a treatment with an IL-6 signaling pathway inhibitor.

Here, each embodiment and a preferred embodiment of the "subject", "IL-6-related disease", "IL-6 signaling pathway inhibitor" and the like in the present embodiment are as described in the section "Biomarker for selecting subject who is likely to have responsiveness to treatment with IL-6 signaling pathway inhibitor".

Further, each embodiment and a preferred embodiment of the "measurement" in the present embodiment are as described in the section "Method for measuring biomarker".

Here, more specifically, regarding at least one biomarker described in the section "Biomarker for selecting subject who is likely to have responsiveness to treatment with IL-6 signaling pathway inhibitor", which would be an indicator of whether or not the subject is likely to have a responsiveness to a treatment with an IL-6 signaling pathway inhibitor, a corresponding data that makes correspondence between the responsiveness to an IL-6 signaling pathway inhibitor and the content of the biomarker may be acquired in advance.

This allows, regarding the subject to be tested, measuring a content of a biomarker and predicting the corresponding responsiveness thereto. Then, based on the prediction of this responsiveness, a subject who is likely to have a responsiveness to a treatment with IL-6 signaling pathway inhibitor can be selected.

Reference value (threshold) of the content of the biomarker, which would be a reference value for the presence or absence of responsiveness, can be set appropriately by those skilled in the art, depending on the degree of the sensitivity and/or the specificity at which the responsiveness is desired to be predicted.

For example, when the subject is a subject suffering from PAH, in other words, the method according to the present embodiment is a method for selecting a subject suffering from PAH who is likely to have a responsiveness to a treatment with IL-6 signaling pathway inhibitor, the threshold of the blood content of the biomarker, which is IL-6, can be suitably selected by those skilled in the art with reference to the threshold, sensitivity and specificity as shown in Figure 8, considering how or to what extent the subject group is to be discriminated by IL-6. For example, the sensitivity and the specificity may be selected so as to maximize the sensitivity and/or the specificity, or maximize the sum of the sensitivity and the specificity.

Specifically, in one embodiment, for the blood content of IL-6, the threshold is preferably a threshold corresponding to the case where both the sensitivity and the specificity are relatively high, for example, preferably a threshold corresponding to the case where the sum of the sensitivity and the specificity is about 1.3 to about 1.6, and more preferably a threshold corresponding to the case where the sum of the sensitivity and the specificity is about 1.4 to about 1.6. The sensitivity and the specificity may then be selected so that either the sensitivity or the specificity is relatively high, considering how or to what extent the subject group is to be discriminated by IL-6.

More specifically, for the serum content of IL-6, the threshold may be about 1.0 to about 5.0 (pg/mL), about 1.5 to about 4.0 (pg/mL), or about 2.0 to about 3.0 (pg/mL). In one embodiment, when referring to Figure 8, the threshold of serum content of IL-6 may be selected as 2.12 (pg/mL), 2.29 (pg/mL), 2.43 (pg/mL), 2.54 (pg/mL), 2.73 (pg/mL), or 2.79 (pg/mL).

Further, for example, when the subject is a subject suffering from PAH, in other words, the method according to the present embodiment is a method for selecting a subject suffering from PAH who is likely to have a responsiveness to a treatment with IL-6 signaling pathway inhibitor, the threshold of the blood content of the biomarker, which is IL-1β, can be suitably selected by those skilled in the art with reference to the threshold, sensitivity and specificity as shown in Figure 9, considering how or to what extent the subject group is to be discriminated by IL-1β. For example, the sensitivity and the specificity may be selected so as to maximize the sensitivity and/or the specificity, or maximize the sum of the sensitivity and the specificity.

Specifically, in one embodiment, for the blood content of IL-1β, the threshold is preferably a threshold corresponding to the case where both the sensitivity and the specificity are high, for example, preferably a threshold corresponding to the case where the sum of the sensitivity and the specificity is about 1.3 to about 1.6, and more preferably a threshold corresponding to the case where the sum of the sensitivity and the specificity is about 1.4 to about 1.6. The sensitivity and the specificity may then be selected so that either the sensitivity or the specificity is relatively high, considering how or to what extent the subject group is to be discriminated by the IL-1β.

More specifically, for the serum content of IL-1β, the threshold may be about 3.0 to about 9.0 (pg/mL), about 3.5 to about 7.0 (pg/mL), or about 4.0 to about 6.0 (pg/mL). In one embodiment, when referring to Figure 9, the threshold of content of IL-1β may be selected as 4.49 (pg/mL), 4.50 (pg/mL), 5.04 (pg/mL), 5.13 (pg/mL), 5.59 (pg/mL), or 5.76 (pg/mL).

= = Method for measuring biomarker = =

The method for measuring a biomarker according to the present embodiment is a measuring method comprising measuring a content of a biomarker comprising one or more cytokines selected from the group consisting of IL-6, CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1 in a sample collected from a subject.

In one embodiment, the measuring method may be a method for measuring a biomarker comprising one or more cytokines or a combination of two or more cytokines selected based on any conditions such as reliability of prediction, application, economy, and/or simplicity of measurement.

For example, when the ease of measurement and/or economy is particularly considered, the measuring method is preferably a method for measuring a biomarker comprising IL-6, IL-1 β/IL-1 F2, or IL-4, and may be a method for measuring a combination of two or three cytokines of these. That is, the measuring method may be a method for measuring a combination of IL-6 and IL-1 β/IL-1F2, a combination of IL-6 and IL-4, a combination of IL-1β/IL-1F2 and IL-4, or a combination of IL-6, IL-1 β/IL-1F2, and IL-4.

By the method for measuring a biomarker according to the present embodiment, at least one biomarker described in the section "Biomarker for predicting content of IL-6 in subject", "Biomarker for predicting responsiveness of subject to treatment with IL-6 signaling pathway inhibitor" and/or "Biomarker for selecting subject who is likely to have responsiveness to treatment with IL-6 signaling pathway inhibitor" can be measured. Thus, by the method for measuring a biomarker according to the present embodiment, data for the purpose of predicting a content of IL-6 in a subject", for the purpose of predicting a responsiveness of a subject to a treatment with an IL-6 signaling pathway inhibitor and/or for the purpose of selecting a subject who is likely to have a responsiveness to a treatment with an IL-6 signaling pathway inhibitor can be collected.

[Subject]

The "subject" in the measurement method can be suitably selected based on the purpose for which the data is collected. However, except the matters specified below, each embodiment and a preferred embodiment are as described in the section "Biomarker for predicting content of IL-6 in subject", "Biomarker for predicting responsiveness of subject to treatment with IL-6 signaling pathway inhibitor" and/or "Biomarker for selecting subject who is likely to have responsiveness to treatment with IL-6 signaling pathway inhibitor".

[Measurement of biomarker in sample]

The sample is a biological sample collected from a subject group, and is not limited as long as it is a sample in which at least one of the biomarkers is present at a content that can be measured. For example, the sample may be a solid tissue sample obtained by a biopsy or during a treatment, or may be blood (whole blood), plasma, or serum.

In one embodiment, when the subject is a human patient suffering from PAH, the sample is preferably blood (whole blood), plasma, or serum, more preferably plasma or serum.

It is preferred that a necessary pretreatment is performed on the sample before being subjected to a measurement. For example, when the sample to be used is plasma or serum, it is preferred that the whole blood is treated by standing still or centrifugation or the like, then plasma or serum is separated from other blood components.

The content of a biomarker that is a cytokine in a sample collected from a subject can be measured using a conventional method used in the art as a method for measuring a cytokine and is not limited as long as the content can be measured.

In one embodiment, the content of the cytokine is preferably measured using a method based on antigen-antibody reaction, for example, using antibodies that specifically bind to each cytokine. Examples of the method based on the antigen-antibody reaction include a Western blot method, an ELISA method (enzyme-linked immunosorbent assay), an FCM method (flow cytometry method), and an antibody-array method, which can be appropriately selected by those skilled in the art according to the purpose of measurement.

In one embodiment, as a method for visualizing and quantifying the antigen-antibody reaction, a conventional method used in the art may be used. For example, a radio isotope method, a fluorescence method, an enzymatic method, and/or a chemiluminescence method may be used as the method for visualization. Furthermore, for example, as a method for quantification, a direct method or an indirect method, or a sensitization method using a biotinavidin complex or a labeled avidin may be used.

Here, the content of the cytokine, which is a biomarker, may be any of an absolute value, a relative concentration, a weight per unit volume, raw data measured to know the absolute concentration, or the like of the biomarker.

In one embodiment, when plasma or serum is measured as a sample, it is preferred that the content is a weight per unit volume of the sample, such as 1 mL or 1 μL.

= = Kit = =

The kit according to the present embodiment is a kit comprising a means for carrying out the measurement method described in the "Method for measuring biomarker".

In one embodiment, the kit preferably comprises a means for measuring at least one of the biomarkers described in the section "Biomarker for predicting content of IL-6 in subject", "Biomarker for predicting responsiveness of subject to treatment with IL-6 signaling pathway inhibitor" and/or "Biomarker for selecting subject who is likely to have responsiveness to treatment with IL-6 signaling pathway inhibitor".

This can be used for predicting a content of IL-6 in a subject, for predicting a responsiveness of a subject to a treatment with an IL-6 signaling pathway inhibitor, and/or for selecting a subject who is likely to have a responsiveness to a treatment with an IL-6 signaling pathway inhibitor.

In the kit, each embodiment and a preferred embodiment of the "subject", "IL-6-related disease", "IL-6 signaling pathway inhibitor" and the like are as described in the section "Biomarker for predicting content of IL-6 in subject", "Biomarker for predicting responsiveness of subject to treatment with IL-6 signaling pathway inhibitor" and/or "Biomarker for selecting subject who is likely to have responsiveness to treatment with IL-6 signaling pathway inhibitor".

In one embodiment, the kit preferably comprises, as a means for carrying out the measurement method described in the "Method for measuring biomarker", an antibody that specifically binds to at least one of the biomarkers described in the section "Biomarker for predicting content of IL-6 in subject", "Biomarker for predicting responsiveness of subject to treatment with IL-6 signaling pathway inhibitor" and/or "Biomarker for selecting subject who is likely to have responsiveness to treatment with IL-6 signaling pathway inhibitor". That is, the kit preferably comprises an antibody that specifically binds to at least one cytokine that is included in the biomarker described in the section "Biomarker for predicting content of IL-6 in subject", "Biomarker for predicting responsiveness of subject to treatment with IL-6 signaling pathway inhibitor" and/or "Biomarker for selecting subject who is likely to have responsiveness to treatment with IL-6 signaling pathway inhibitor".

By using such an antibody, the content of cytokine in a sample can be measured by a method based on an antigen-antibody reaction, such as a Western blot method, an ELISA method (enzyme-linked immunosorbent assay), or an antibody-array method.

In one embodiment, the kit may comprise a reagent for visualizing and quantifying the antigen-antibody reaction, such as a radioisotope, a fluorescent dye, an enzyme, a bead, and/or a chemiluminescent substrate.

In one embodiment, the kit may contain various buffers necessary for measuring a biomarker, or a concentrate thereof.

In one embodiment, the kit may comprise a standard biomarker sample.

In one embodiment, the kit may comprise a container for storing or processing a sample collected from a subject.

= = Medicament, treating method, IL-6 signaling pathway inhibitor, and use of IL-6 signaling pathway inhibitor = =

The subject selected by "Method for selecting subject who is likely to have responsiveness to treatment with IL-6 signaling pathway inhibitor" is likely to have a responsiveness to a treatment with an IL-6 signaling pathway inhibitor.

Accordingly, the method can exclude in advance a subject who is likely to have no or few effects of a treatment with an IL-6 signaling pathway inhibitor, and can apply a treatment with an IL-6 signaling pathway inhibitor only to a subject who is likely to have a responsiveness to the treatment, which is effective in personalized medicine.

The medicament according to the present embodiment is a medicament comprising an IL-6 signaling pathway inhibitor, which is administered to a subject selected as a subject who is likely to have a responsiveness to a treatment with an IL-6 signaling pathway inhibitor by the selecting method described in the "Method for selecting subject who is likely to have responsiveness to treatment with IL-6 signaling pathway inhibitor".

The treating method according to another embodiment is a treating method comprising administering a therapeutically effective amount of an IL-6 signaling pathway inhibitor to a subject selected as a subject who is likely to have a responsiveness to a treatment with an IL-6 signaling pathway inhibitor by the selecting method described in the "Method for selecting subject who is likely to have responsiveness to treatment with IL-6 signaling pathway inhibitor".

As used herein, the term "therapeutically effective amount" refers to an amount in which any favorable outcome is consecutively or temporarily obtained in a subject to be administered. Examples of the "favorable outcome" include suppression or improvement of a systemic symptom in a subject, suppression or improvement of pathological symptoms, suppression or improvement of test data reflecting a disease condition, and/or improvement of prognosis of a subject.

The IL-6 signaling pathway inhibitor according to another embodiment is an IL-6 signaling pathway inhibitor, which is administered to a subject selected as a subject who is likely to have a responsiveness to a treatment with an IL-6 signaling pathway inhibitor by the selecting method described in the "Method for selecting subject who is likely to have responsiveness to treatment with IL-6 signaling pathway inhibitor".

The use according to another embodiment is use of an IL-6 signaling pathway inhibitor in producing of a medicament that is administered to a subject selected as a subject who is likely to have a responsiveness to a treatment with an IL-6 signaling pathway inhibitor by the selecting method described in the "Method for selecting subject who is likely to have responsiveness to treatment with IL-6 signaling pathway inhibitor".

In each embodiment of the above-described "medicament," "treating method" "IL-6 signaling pathway inhibitor," and/or "use of an IL-6 signaling pathway inhibitor," each embodiment and a preferred embodiment of the "subject", "IL-6-related disease", "IL-6 signaling pathway inhibitor" and the like are as described in the section "Method for selecting subject who is likely to have responsiveness to treatment with IL-6 signaling pathway inhibitor".

That is, a preferred embodiment of the "medicament" includes a medicament for treating PAH, comprising an IL-6 signaling pathway inhibitor, which is administered to a subject suffering from PAH, selected as a subject who is likely to have a responsiveness to a treatment with an IL-6 signaling pathway inhibitor by the selecting method described in the "Method for selecting subject who is likely to have responsiveness to treatment with IL-6 signaling pathway inhibitor".

PAH can also be treated by inhibition of action of IL-6. Thus, the IL-6 signaling pathway inhibitor is effective in treating PAH.

Another embodiment of the "medicament" includes a medicament for treating PAH, comprising an IL-6 signaling pathway inhibitor.

Another embodiment of the "medicament" includes a medicament for treating PAH, comprising an IL-6 signaling pathway inhibitor administered to a subject suffering from PAH who has a serum content of IL-6 of 2.73 (pg/mL) or more.

The serum content of IL-6 can be measured, for example, by the method described in the section [Measurement of biomarker in sample] in "Method for measuring biomarker".

Here, the "IL-6 signaling pathway inhibitor" in the medicament is as described in the section [IL-6 signaling pathway inhibitor] in "Explanation of terms", but preferably an antibody having an IL-6 signaling pathway inhibitory function, and more preferably an anti-IL-6 receptor antibody or an antigen-binding fragment thereof.

In one embodiment, examples of the anti-IL-6 receptor include an antibody that contains at least one of three hypervariable regions (CDRs) in a heavy chain variable region amino acid sequence having SEQ ID NO: 1 and/or at least one of three hypervariable regions (CDRs) in a light chain variable region amino acid sequence having SEQ ID NO: 2.

In one embodiment, examples of the anti-IL-6 receptor include an antibody that contains a heavy chain containing an amino acid sequence of SEQ ID NO: 3 and a light chain containing an amino acid sequence of SEQ ID NO: 4.

In one embodiment, examples of the anti-IL-6 receptor antibody include satralizumab, tocilizumab, and/or sarilumab, but satralizumab and/or tocilizumab are preferred, and satralizumab is more preferred.

In one embodiment, the medicament may be a medicament administered concurrently, sequentially, or separately and used in combination with other therapeutic agents used in a treatment of PAH, and may be formulated into a single formulation with such therapeutic agents.

The medicament may also be formulated into the form of a tablet, a granule, a powder, a capsule, an emulsion, a suspension, a syrup, or an injection such as a sterile solution or suspension, containing any pharmacologically acceptable carrier, excipient, or stabilizer.

That is, a preferred embodiment of the "treating method" includes a treating method of PAH, comprising administering a therapeutically effective amount of an IL-6 signaling pathway inhibitor to a subject suffering from PAH, selected as a subject who is likely to have a responsiveness to a treatment targeting an IL-6 related cell signaling system by the selecting method described in the "Method for selecting subject who is likely to have responsiveness to treatment with IL-6 signaling pathway inhibitor".

Another embodiment of the "treating method" includes a method for treating PAH, comprising administering to a subject suffering from PAH in need of treatment a therapeutically effective amount of an IL-6 signaling pathway inhibitor.

Another embodiment of the "treating method" includes a method for treating PAH, comprising administering an IL-6 signaling pathway inhibitor to a subject suffering from PAH in need of treatment who has a serum content of IL-6 of 2.73 (pg/mL) or more.

The serum content of IL-6 can be measured, for example, by the method described in the section [Measurement of biomarker in sample] in "Method for measuring biomarker".

Here, the "IL-6 signaling pathway inhibitor" in the treating method is as described in the section [IL-6 signaling pathway inhibitor] in "Explanation of terms", but preferably an antibody having an IL-6 signaling pathway inhibitory function, and more preferably an anti-IL-6 receptor antibody or an antigen-binding fragment thereof.

In one embodiment, examples of the anti-IL-6 receptor include an antibody that contains at least one of three hypervariable regions (CDRs) in a heavy chain variable region amino acid sequence having SEQ ID NO: 1 and/or at least one of three hypervariable regions (CDRs) in a light chain variable region amino acid sequence having SEQ ID NO: 2.

In one embodiment, examples of the anti-IL-6 receptor include an antibody that contains a heavy chain containing an amino acid sequence of SEQ ID NO: 3 and a light chain containing an amino acid sequence of SEQ ID NO: 4.

In one embodiment, examples of the anti-IL-6 receptor antibody include satralizumab, tocilizumab, and/or sarilumab, but satralizumab and/or tocilizumab are preferred, and satralizumab is more preferred.

In one embodiment, in the treating method, a therapeutically effective amount of an IL-6 signal inhibitor may be administered concurrently, sequentially, or separately and used in combination with other therapeutic agents used in a treatment of PAH, and may be formulated into a single formulation with such therapeutic agents.

That is, a preferred embodiment of the "IL-6 signaling pathway inhibitor" includes an IL-6 signaling pathway inhibitor for use in a treatment of PAH, wherein the medicament is administered to a subject suffering from PAH, selected as a subject who is likely to have a responsiveness to a treatment with an IL-6 signaling pathway inhibitor by the selecting method described in the "Method for selecting subject who is likely to have responsiveness to treatment with IL-6 signaling pathway inhibitor".

Another embodiment of the "IL-6 signaling pathway inhibitor" includes an IL-6 signaling pathway inhibitor for use in a treatment of PAH.

Another embodiment of the "IL-6 signaling pathway inhibitor" includes an IL-6 signaling pathway inhibitor for use in treating PAH in a subject suffering from PAH who has a serum content of IL-6 of 2.73 (pg/mL) or more.

The serum content of IL-6 can be measured, for example, by the method described in the section [Measurement of biomarker in sample] in "Method for measuring biomarker".

Here, the "IL-6 signaling pathway inhibitor" is as described in the section [IL-6 signaling pathway inhibitor] in "Explanation of terms", but preferably an antibody having an IL-6 signaling pathway inhibitory function, and more preferably an anti-IL-6 receptor antibody or an antigen-binding fragment thereof.

In one embodiment, examples of the anti-IL-6 receptor include an antibody that contains at least one of three hypervariable regions (CDRs) in a heavy chain variable region amino acid sequence having SEQ ID NO: 1 and/or at least one of three hypervariable regions (CDRs) in a light chain variable region amino acid sequence having SEQ ID NO: 2.

In one embodiment, examples of the anti-IL-6 receptor include an antibody that contains a heavy chain containing an amino acid sequence of SEQ ID NO: 3 and a light chain containing an amino acid sequence of SEQ ID NO: 4.

In one embodiment, examples of the anti-IL-6 receptor antibody include satralizumab, tocilizumab, and/or sarilumab, but satralizumab and/or tocilizumab are preferred, and satralizumab is more preferred.

In one embodiment, the IL-6 signaling pathway inhibitor may be administered concurrently, sequentially, or separately and used in combination with other therapeutic agents used in a treatment of PAH, and may be used in a state formulated into a single formulation with such therapeutic agents.

A preferred embodiment of the "use" includes use of an IL-6 signaling pathway inhibitor in producing of a medicament for treating PAH, wherein the medicament is administered to a subject suffering from PAH, selected as a subject who is likely to have a responsiveness to a treatment with an IL-6 signaling pathway inhibitor by the selecting method described in the "Method for selecting subject who is likely to have responsiveness to treatment with IL-6 signaling pathway inhibitor".

Another embodiment of the "use" includes use of an IL-6 signaling pathway inhibitor in producing of a medicament for treating PAH.

Another embodiment of the "use" includes use of an IL-6 signaling pathway inhibitor in producing of a medicament for treating PAH, wherein the medicament is administered to a subject suffering from PAH who has a serum content of IL-6 of 2.73 (pg/mL) or more.

The serum content of IL-6 can be measured, for example, by the method described in the section [Measurement of biomarker in sample] in "Method for measuring biomarker".

Here, the "IL-6 signaling pathway inhibitor" in the use is as described in the section [IL-6 signaling pathway inhibitor] in "Explanation of terms", but preferably an antibody having an IL-6 signaling pathway inhibitory function, and more preferably an anti-IL-6 receptor antibody or an antigen-binding fragment thereof.

In one embodiment, examples of the anti-IL-6 receptor include an antibody that contains at least one of three hypervariable regions (CDRs) in a heavy chain variable region amino acid sequence having SEQ ID NO: 1 and/or at least one of three hypervariable regions (CDRs) in a light chain variable region amino acid sequence having SEQ ID NO: 2.

In one embodiment, examples of the anti-IL-6 receptor include an antibody that contains a heavy chain containing an amino acid sequence of SEQ ID NO: 3 and a light chain containing an amino acid sequence of SEQ ID NO: 4.

In one embodiment, examples of the anti-IL-6 receptor antibody include satralizumab, tocilizumab, and/or sarilumab, but satralizumab and/or tocilizumab are preferred, and satralizumab is more preferred.

In one embodiment, the use may be use in producing of a medicament administered concurrently, sequentially, or separately and used in combination with other therapeutic agents used in a treatment of PAH. In this case, the medicament may be formulated into a single formulation with such therapeutic agents.

[Examples]

Hereinafter, embodiments of the present invention completed based on the above-described findings are described in detail with reference to Examples. The objects, features, advantages, and ideas of the present invention are clear to those skilled in the art by the description herein, and those skilled in the art can easily reproduce the present invention from the description herein. Embodiments of the present invention and specific examples thereof described below are indicative of preferred embodiments of the present invention and shown for illustration or description, thus the present invention is not limited to these. It will be apparent to those skilled in the art that various changes and modifications can be made within the intent and scope of the present invention disclosed herein, based on the description herein.

[Example 1] Measurement of biomarker candidate substances

The serums of 145 patients, who are patients with pulmonary arterial hypertension in six institutions (International University of Health and Welfare, Mita Hospital; Chiba University Hospital, Nippon Medical School Hospital, Kyorin University Hospital, Kyushu University Hospital, Kobe University Hospital) among the participating institutions of the pulmonary hypertension patient registry (JAPHR, www.japanph.com/japhr), and who had a sample measurement in January 2021 in this study and gave opt-out consent for this study, were acquired.

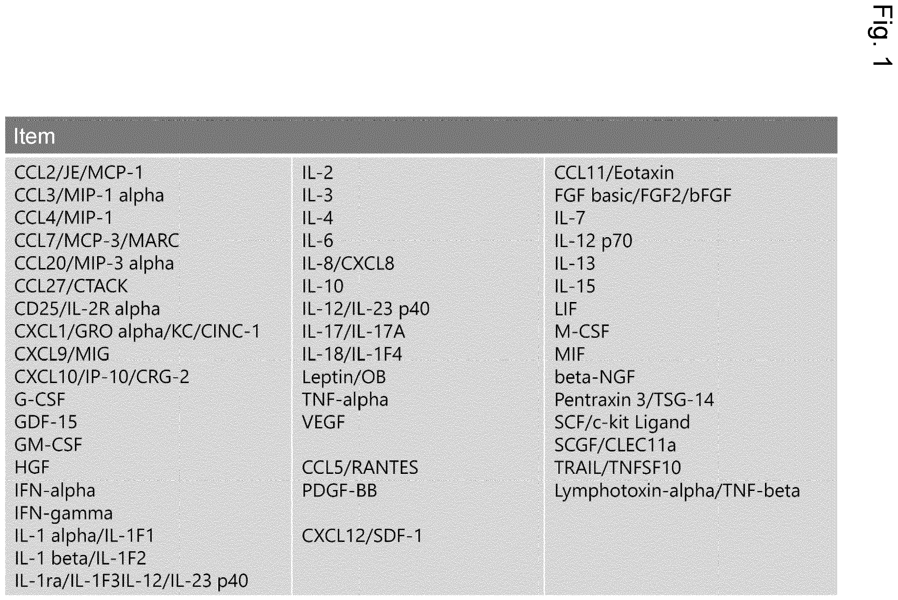

For each of the 145 specimens, the contents of 49 cytokines (Figure 1) were measured by contract analysis with Filgen, Inc., using the Human Magnetic Luminex Assay kit (R&D Systems, Inc.).

Of the 49 cytokines, IL-12 p70, LIF, and Lymphotoxin-alpha/TNF-beta could not be measured due to being below the detection limit, and measurement results were obtained for 46 cytokines including IL-6.

[Example 2] Clustering of biomarker candidate substances

= = Preprocessing of measurement data = =

The content of each cytokine measured in Example 1 was taken as 0 (zero) in the case that it was equal to or less than the measurement sensitivity (OOR), and the top 5 of the 145 data were taken as 5th value from the top.

= = Acquisition of cosine similarity = =

Data for each content of cytokines other than IL-6, obtained as described above, were arranged to form vectors of 145 specimens, i.e., 145-dimensional vectors. The vectors thus acquired are believed to represent the properties of each cytokine.

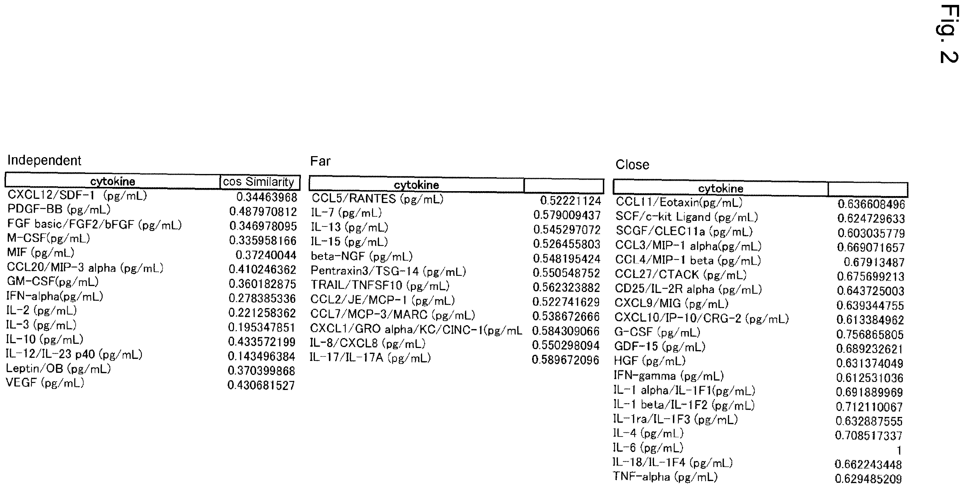

Subsequently, cosine similarity was calculated for IL-6 and each of the cytokines other than IL-6 (45 cytokines) using the following formula. The results are shown in Figure 2.

$$\frac{\vec{x} \cdot \vec{y}}{|\vec{x}||\vec{y}|} = \frac{x_1 y_1 + x_2 y_2 + \cdots + x_n y_n}{\sqrt{x_1^2 + x_2^2 + \cdots + x_n^2}\sqrt{y_1^2 + y_2^2 + \cdots + y_n^2}}$$

In the above formula, x was the content of IL-6, y was another cytokine, and n was the number of data (145 specimens).

The cosine similarity corresponds to $\cos\theta$ when the angle between the vector of x and the vector of y is taken as $\theta$. It can be said that the closer the cosine similarity is to 1, the closer the vector of x (vector of content of IL-6) and the vector of y (vector of another cytokine) are to the same direction. That is, the vector similarity indicates the similarity of behaviors of IL-6 and each of the other cytokines.

= = Classification of cytokines based on cosine similarity = =

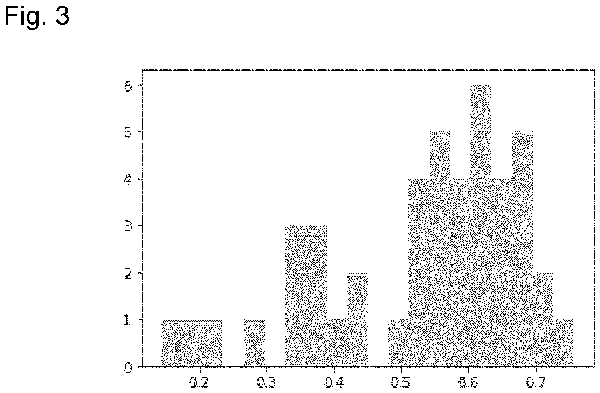

A histogram created using the cosine similarity for IL-6 and each of the other cytokines acquired as described above is shown in Figure 3.

Based on Figure 3, cytokines were able to be classified into a cytokine group independent of IL-6 ("Independent" in Figure 2) with a cosine similarity of about 0.5 or less, and cytokine groups having a relation with IL-6 ("Close" and "Far" in Figure 2) with a cosine similarity of higher than about 0.5.

For the cytokine group having a relation with IL-6, the first selection of biomarker candidates was performed by taking the cytokines with cosine similarity of 0.6 or more ("Close" in Figure 2) as the IL-6 similar group.

The cytokines other than IL-6 included in the IL-6 similar group were 19 cytokines, i.e., 1L-1α/IL-1F1, IL-1β/IL-1F2, IL-4, IL-18/1L-1F4, IL-1RA/IL-1F3, TNF-α, IFN-γ, GDF-15, CCL3/MIP-1α, CCL4/MIP-1β, G-CSF, CXCL9/MIG, CXCL10/IP-10/CRG-2, CCL27/CTACK, SCF/c-kit Ligand, SCGF/CLEC11a, CD25/IL-2Rα, HGF, and CCL11/Eotaxin.

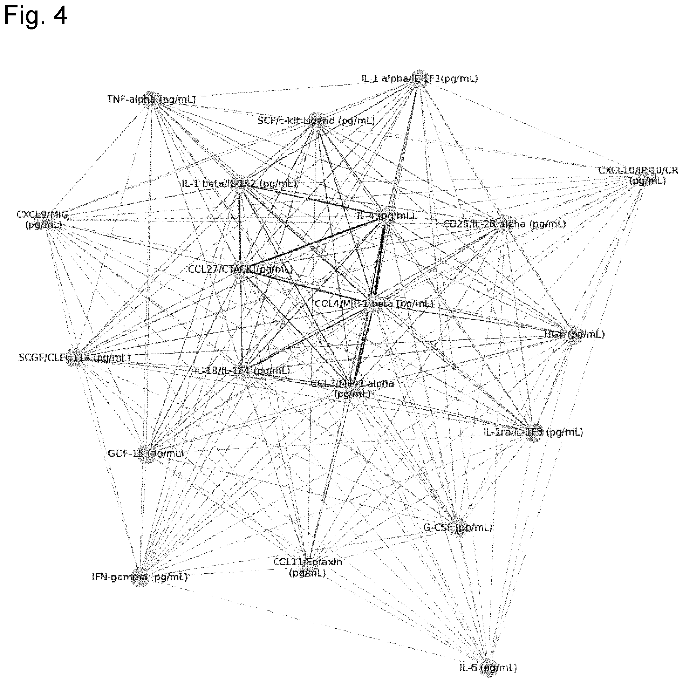

Figure 4 shows a network graph created based on the cosine similarity with IL-6 for each cytokine included in the IL-6 similar group. In Figure 4, the thickness of the side of the graph corresponds to the magnitude of the cosine similarity.

= = Clustering by k-medoids method = =

Based on the content of the cytokine contained in the IL-6 similar group, 145 specimens were clustered into three clusters using k-medoids method.

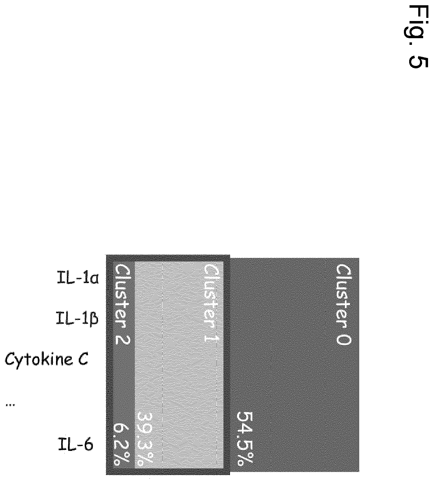

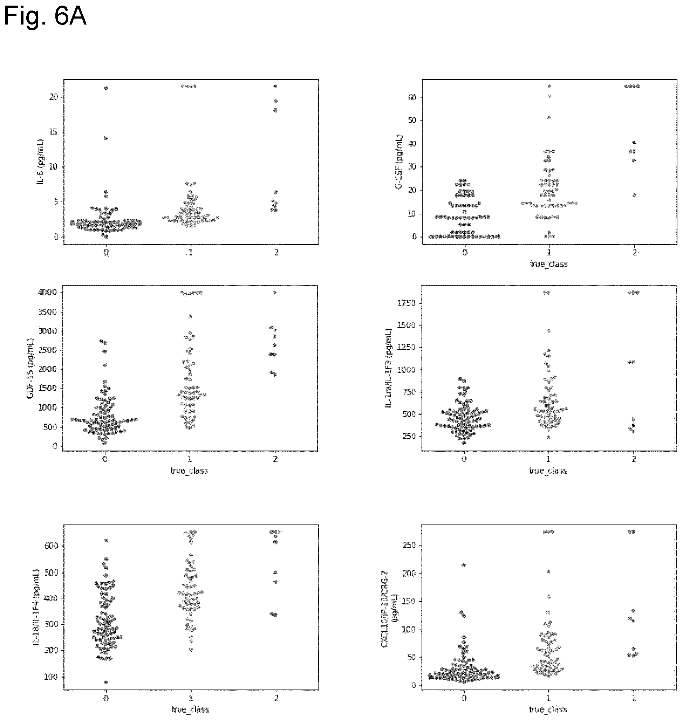

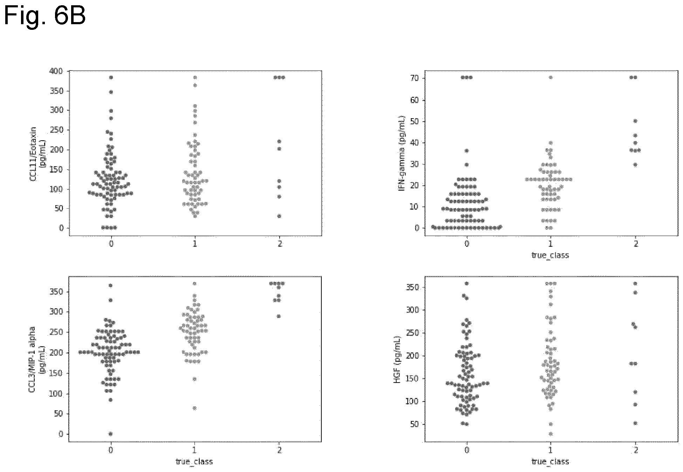

With this clustering, the specimens were clustered into cluster 0, cluster 1, and cluster 2, as shown in Figure 5. Figures 6 (A, B) and 7 (A, B) are graphs showing distribution of the three clusters for each cytokine. In each graph, scale 0 (zero) in the horizontal axis indicates cluster 0, scale 1 indicates cluster 1, and scale 2 indicates cluster 2.

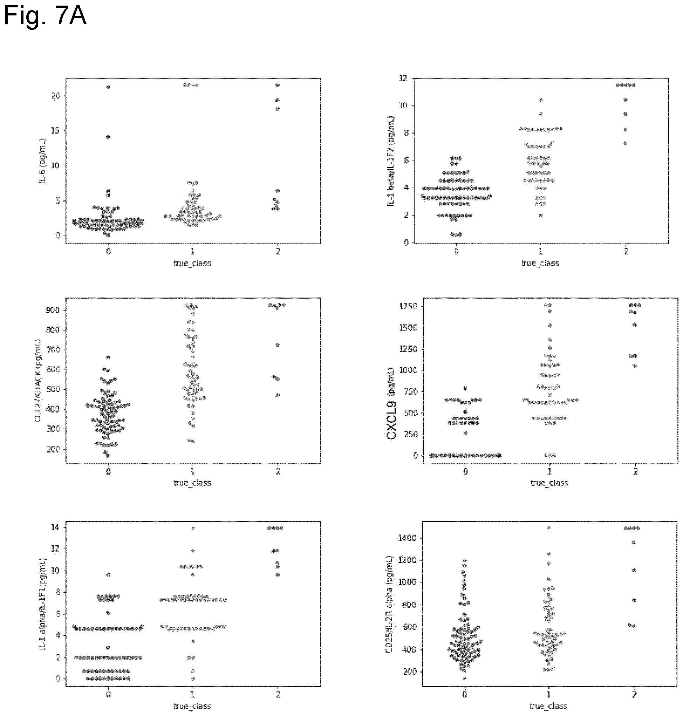

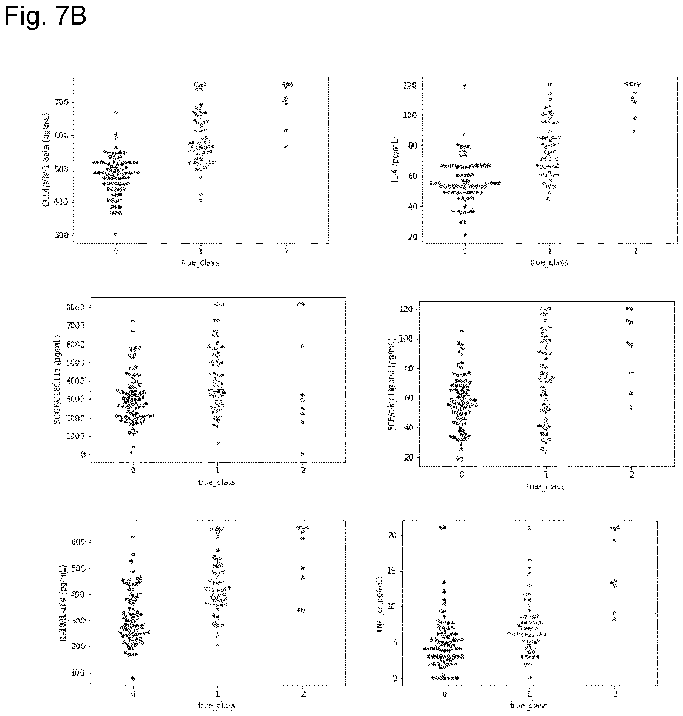

Figure 6 (A, B) shows the clustering results of cytokines located relatively close to IL-6 in the network graph of Figure 4. Figure 7 (A, B) shows the clustering results of cytokines located relatively close to IL-1β in the network graph of Figure 4.

[Example 3] Selection of biomarker candidates based on reliability (AUC)

For each cytokine included in the IL-6 similar group, a series of any threshold candidate values (e.g., corresponding to the left-most column values in Figures 8 and 9) was set for discriminating the specimen groups of clusters 1 and 2 from the specimen group of cluster 0.

For each cytokine, a sensitivity and a specificity were calculated based on whether or not it can be classified into the group including clusters 1 and 2 or the group of cluster 0 according to the k-medoids method described above when discriminated by each of the threshold candidate values thereof.

As examples of this, the corresponding table of the threshold, sensitivity, and specificity in IL-6 is shown in Figure 8, and the corresponding table of the threshold, sensitivity, and specificity in IL-1β is shown in Figure 9.

For each cytokine included in the IL-6 similar group, a receiver operating curve (ROC) was drawn using the sensitivity and the specificity acquired in the same way to acquire an area under the curve (AUC).

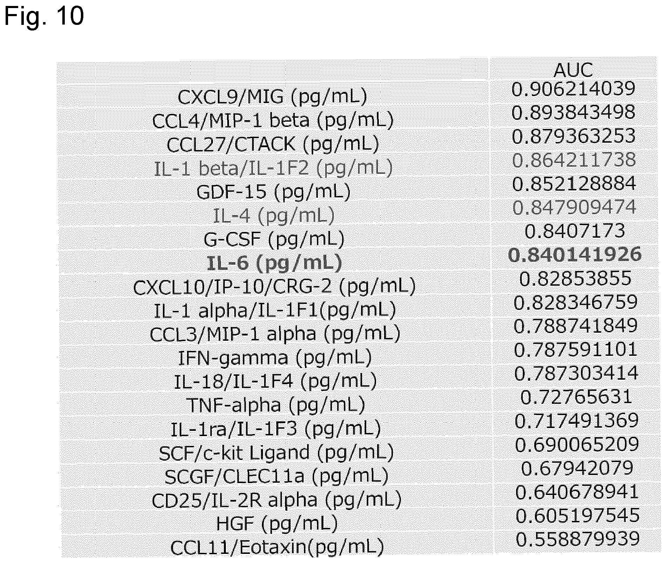

AUC is an indicator, for each cytokine, showing the reliability of the function of classifying the specimen groups of clusters 1 and 2 from the specimen group of cluster 0. Figure 10 shows the AUC of each cytokine included in the IL-6 similar group.

In general, when the AUC is greater than or equal to about 0.8, the cytokine is determined to be useful as a marker.

From the above, it was shown that CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1 are cytokines that exhibit similar behavior to IL-6. That is, they are indicators of IL-6 behavior in patients.

The threshold value when using these cytokines as markers for discriminating the high IL-6 content group and the low IL-6 content group can be determined based on the sensitivity and the specificity.

With reference to Figures 8 and 9, the threshold could be appropriately set, for example, for IL-6 with consideration of how the patient should be discriminated, from the threshold corresponding to the sensitivity and the specificity at which the sum is relatively high of about 1.4 to about 1.6. In particular, the threshold of serum content of IL-6 at which the sum of the sensitivity and the specificity is near about 1.5 was 2.12 (pg/mL), 2.29 (pg/mL), 2.43 (pg/mL), 2.54 (pg/mL), 2.73 (pg/mL), or 2.79 (pg/mL).

The threshold could be appropriately set, for example, for IL-1β with consideration of how the patient should be discriminated, from the threshold corresponding to the sensitivity and the specificity at which the sum is relatively high of about 1.4 to about 1.6. In particular, the threshold of serum content of IL-1β at which the sum of the sensitivity and the specificity is about 1.5 or more was 4.49 (pg/mL), 4.50 (pg/mL), 5.04 (pg/mL), 5.13 (pg/mL), 5.59 (pg/mL), or 5.76 (pg/mL).

As described in Background Art, it has been reported that IL-6 in blood is elevated and expression of IL-6 receptors in diseased organs is high in PAH, and that treatments targeting IL-6 signaling pathway are effective in pulmonary arterial hypertension. IL-6 in blood in patients and/or IL-6 receptor in an organ are a direct target for treatment with an IL-6 signaling pathway inhibitor such as an anti-IL-6 antibody or an anti-IL-6 receptor antibody. That is, as described above, it is recognized that pulmonary arterial hypertension can be treated by inhibition of IL-6 action, and the content of IL-6 in a patient with pulmonary arterial hypertension would be an indicator of responsiveness to a treatment with an IL-6 signaling pathway inhibitor.

Thus, IL-6, CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1 are indicators for predicting a responsiveness to a treatment with an IL-6 signaling pathway inhibitor in a patient suffering from an IL-6-related disease, especially PAH.

**Claims**

1. A biomarker for predicting a content of IL-6 in a subject suffering from PAH, the biomarker comprising one or more cytokines selected from the group consisting of CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1.

2. A biomarker for predicting a responsiveness of a subject suffering from PAH to a treatment with an IL-6 signaling pathway inhibitor, the biomarker comprising
one or more cytokines selected from the group consisting of CXCL9/MIG, CCL4/MIP-1β, CCL27/CTACK, IL-1β/IL-1F2, GDF-15, IL-4, G-CSF, IL-6, CXCL10/IP-10/CRG-2, and IL-1α/IL-1F1.

3. A biomarker for selecting a subject suffering from PAH who is likely to have a responsiveness to a treatment with an IL-6 signaling pathway inhibitor, the biomarker comprising
one or more cytokines selected from the group consisting of CXCL9/MIG, CCL4/MIP-1β, CCL27/CTACK, IL-1β/IL-1F2, GDF-15, IL-4, G-CSF, IL-6, CXCL10/IP-10/CRG-2, and IL-1α/IL-1F1.

4. Use of a biomarker for predicting a content of IL-6 in a subject suffering from PAH, the biomarker comprising one or more cytokines selected from the group consisting of CXCL9/MIG, CCL4/MIP-1β, CCL27/CTACK, IL-1β/IL-1F2, GDF-15, IL-4, G-CSF, CXCL10/IP-10/CRG-2, and IL-1α/IL-1F1.

5. Use of a biomarker for predicting a responsiveness of a subject suffering from PAH to a treatment with an IL-6 signaling pathway inhibitor, the biomarker comprising one or more cytokines selected from the group consisting of CXCL9/MIG, CCL4/MIP-1β, CCL27/CTACK, IL-1β/IL-1F2, GDF-15, IL-4, G-CSF, IL-6, CXCL10/IP-10/CRG-2, and IL-1α/IL-1F1.

6. Use of a biomarker for selecting a subject suffering from PAH who is likely to have a responsiveness to a treatment with an IL-6 signaling pathway inhibitor, the biomarker comprising one or more cytokines selected from the group consisting of CXCL9/MIG, CCL4/MIP-1β, CCL27/CTACK, IL-1β/IL-1F2, GDF-15, IL-4, G-CSF, IL-6, CXCL10/IP-10/CRG-2, and 1L-1α/IL-1F1.

7. A method for predicting a content of IL-6 in a subject suffering from PAH, comprising measuring a content of the biomarker according to claim 1 in a sample collected from the subject.

8. A method for predicting a responsiveness of a subject suffering from PAH to a treatment with an IL-6 signaling pathway inhibitor, comprising:

measuring a content of the biomarker according to claim 2 in a sample collected from the subject; and
determining, based on the content of the biomarker, whether or not the subject is likely to have a responsiveness to a treatment with an IL-6 signaling pathway inhibitor.

9. A method for selecting a subject suffering from PAH who is likely to have a responsiveness to a treatment with an IL-6 signaling pathway inhibitor, comprising

measuring a content of the biomarker according to claim 3 in a sample collected from the subject; and

determining, based on the content of the biomarker, whether or not the subject is likely to have a responsiveness to a treatment with an IL-6 signaling pathway inhibitor.

10. A kit used for predicting a content of IL-6 in a subject suffering from PAH, comprising a means for measuring the biomarker according to claim 1.

11. A kit used for predicting a responsiveness of a subject suffering from PAH to a treatment with an IL-6 signaling pathway inhibitor, comprising a means for measuring the biomarker according to claim 2.

12. A kit used for selecting a subject suffering from PAH who is likely to have a responsiveness to a treatment with an IL-6 signaling pathway inhibitor, comprising a means for measuring the biomarker according to claim 3.

13. A medicament for treating PAH, comprising an IL-6 signaling pathway inhibitor, wherein the medicament is administered to a subject suffering from PAH, selected as a subject who is likely to have a responsiveness to a treatment with the IL-6 signaling pathway inhibitor by the selecting method according to claim 9.

14. A method for selecting a novel biomarker, comprising:

(a) selecting a biomarker candidate substance based on a vector similarity acquired using a content of a reference biomarker and a content of the biomarker candidate substance in a sample collected from a subject group; and
(b) clustering the subject group based on the content of the biomarker candidate substance selected in (a).

15. A method for selecting a biomarker for predicting a content of IL-6 in a subject suffering from PAH, comprising:

(a) selecting a biomarker candidate substance based on a vector similarity acquired using a content of IL-6 and a content of the biomarker candidate substance in a sample collected from a subject group suffering from PAH; and
(b) clustering the subject group based on the content of the biomarker candidate substance selected in (a).

16. A method for selecting a biomarker for predicting a responsiveness of a subject suffering from PAH to a treatment with an IL-6 signaling pathway inhibitor, comprising:

(a) selecting a biomarker candidate substance based on a vector similarity acquired using a content of IL-6 and a content of the biomarker candidate substance in a sample collected from a subject group suffering from PAH; and
(b) clustering the subject group based on the content of the biomarker candidate substance selected in (a).

17. A method for selecting a biomarker for selecting a subject suffering from PAH who is likely to have a responsiveness to a treatment with an IL-6 signaling pathway inhibitor, comprising:

(a) selecting a biomarker candidate substance based on a vector similarity acquired using a content of IL-6 and a content of the biomarker candidate substance in a sample collected from a subject group suffering from PAH; and
(b) clustering the subject group based on the content of the biomarker candidate substance selected in (a).

18. A device for selecting a novel biomarker, comprising:

(a) a selection unit that selects a biomarker candidate substance based on a vector similarity acquired using a content of a reference biomarker and a content of the biomarker candidate substance in a sample collected from a subject group; and
(b) a clustering unit that clusters the subject group based on the content of the biomarker candidate substance selected in (a).

19. A device for selecting a biomarker for predicting a content of IL-6 in a subject suffering from PAH, comprising:

(a) a selection unit that selects a biomarker candidate substance based on a vector similarity acquired using a content of IL-6 and a content of the biomarker candidate substance in a sample collected from a subject group suffering from PAH; and
(b) a clustering unit that clusters the subject group based on the content of the biomarker candidate substance selected in (a).

20. A device for selecting a biomarker for predicting a responsiveness of a subject suffering from PAH to a treatment with an IL-6 signaling pathway inhibitor, comprising:

> (a) a selection unit that selects a biomarker candidate substance based on a vector similarity acquired using a content of IL-6 and a content of the biomarker candidate substance in a sample collected from a subject group suffering from PAH; and
> (b) a clustering unit that clusters the subject group based on the content of the biomarker candidate substance selected in (a).

21. A device for selecting a biomarker for selecting a subject suffering from PAH who is likely to have a responsiveness to a treatment with an IL-6 signaling pathway inhibitor, comprising:

> (a) a selection unit that selects a biomarker candidate substance based on a vector similarity acquired using a content of IL-6 and a content of the biomarker candidate substance in a sample collected from a subject group suffering from PAH; and
> (b) a clustering unit that clusters the subject group based on the content of the biomarker candidate substance selected in (a).

Fig. 1

EP 4 321 176 A1

| Item | | |
|---|---|---|
| CCL2/JE/MCP-1 | IL-2 | CCL11/Eotaxin |
| CCL3/MIP-1 alpha | IL-3 | FGF basic/FGF2/bFGF |
| CCL4/MIP-1 | IL-4 | IL-7 |
| CCL7/MCP-3/MARC | IL-6 | IL-12 p70 |
| CCL20/MIP-3 alpha | IL-8/CXCL8 | IL-13 |
| CCL27/CTACK | IL-10 | IL-15 |
| CD25/IL-2R alpha | IL-12/IL-23 p40 | LIF |
| CXCL1/GRO alpha/KC/CINC-1 | IL-17/IL-17A | M-CSF |
| CXCL9/MIG | IL-18/IL-1F4 | MIF |
| CXCL10/IP-10/CRG-2 | Leptin/OB | beta-NGF |
| G-CSF | TNF-alpha | Pentraxin 3/TSG-14 |
| GDF-15 | VEGF | SCF/c-kit Ligand |
| GM-CSF | | SCGF/CLEC11a |
| HGF | CCL5/RANTES | TRAIL/TNFSF10 |
| IFN-alpha | PDGF-BB | Lymphotoxin-alpha/TNF-beta |
| IFN-gamma | | |
| IL-1 alpha/IL-1F1 | CXCL12/SDF-1 | |
| IL-1 beta/IL-1F2 | | |
| IL-1ra/IL-1F3IL-12/IL-23 p40 | | |

## Fig. 2

**Independent**

| cytokine | cos Similarity |
|---|---|
| CXCL12/SDF-1 (pg/mL) | 0.34463968 |
| PDGF-BB (pg/mL) | 0.487970812 |
| FGF basic/FGF2/bFGF (pg/mL) | 0.346978095 |
| M-CSF(pg/mL) | 0.335958166 |
| MIF (pg/mL) | 0.37240044 |
| CCL20/MIP-3 alpha (pg/mL) | 0.410246362 |
| GM-CSF(pg/mL) | 0.360182875 |
| IFN-alpha(pg/mL) | 0.278385336 |
| IL-2 (pg/mL) | 0.221258362 |
| IL-3 (pg/mL) | 0.195347851 |
| IL-10 (pg/mL) | 0.433572199 |
| IL-12/IL-23 p40 (pg/mL) | 0.143496384 |
| Leptin/OB (pg/mL) | 0.3703998868 |
| VEGF (pg/mL) | 0.430681527 |

**Far**

| cytokine | |
|---|---|
| CCL5/RANTES (pg/mL) | 0.52221124 |
| IL-7 (pg/mL) | 0.579009437 |
| IL-13 (pg/mL) | 0.545297072 |
| IL-15 (pg/mL) | 0.526455803 |
| beta-NGF (pg/mL) | 0.548195424 |
| Pentraxin3/TSG-14 (pg/mL) | 0.550548752 |
| TRAIL/TNFSF10 (pg/mL) | 0.562323882 |
| CCL2/JE/MCP-1 (pg/mL) | 0.522741629 |
| CCL7/MCP-3/MARC (pg/mL) | 0.538672666 |
| CXCL1/GRO alpha/KC/CINC-1(pg/mL) | 0.584309066 |
| IL-8/CXCL8 (pg/mL) | 0.550298094 |
| IL-17/IL-17A (pg/mL) | 0.589672096 |

**Close**

| cytokine | |
|---|---|
| CCL11/Eotaxin(pg/mL) | 0.636608496 |
| SCF/c-kit Ligand (pg/mL) | 0.624729633 |
| SCGF/CLEC11a (pg/mL) | 0.603035779 |
| CCL3/MIP-1 alpha(pg/mL) | 0.669071657 |
| CCL4/MIP-1 beta (pg/mL) | 0.67913487 |
| CCL27/CTACK (pg/mL) | 0.675699213 |
| CD25/IL-2R alpha (pg/mL) | 0.643725003 |
| CXCL9/MIG (pg/mL) | 0.639344755 |
| CXCL10/IP-10/CRG-2 (pg/mL) | 0.613384962 |
| G-CSF (pg/mL) | 0.756865805 |
| GDF-15 (pg/mL) | 0.689232621 |
| HGF (pg/mL) | 0.631374049 |
| IFN-gamma (pg/mL) | 0.612531036 |
| IL-1 alpha/IL-1F1(pg/mL) | 0.691889969 |
| IL-1 beta/IL-1F2 (pg/mL) | 0.712110067 |
| IL-1ra/IL-1F3 (pg/mL) | 0.632887555 |
| IL-4 (pg/mL) | 0.708517337 |
| IL-6 (pg/mL) | 1 |
| IL-18/IL-1F4 (pg/mL) | 0.662243448 |
| TNF-alpha (pg/mL) | 0.629485209 |

Fig. 3

Fig. 4

Fig. 5

Fig. 6A

Fig. 6B

Fig. 7A

Fig. 7B

## Fig. 8

Sensitivity and specificity for identifying clusters 1 + 2

| threshold1+2 | sensitivity1+2 | specificity1+2 |
|---|---|---|
| 22.43 | 1 | 0 |
| 21.43 | 1 | 0.075757576 |
| 21.16 | 0.987341772 | 0.075757576 |
| 18.01 | 0.987341772 | 0.106060606 |
| 14.05 | 0.974683544 | 0.106060606 |
| 7.51 | 0.974683544 | 0.136363636 |
| 7.36 | 0.974683544 | 0.151515152 |
| 6.33 | 0.962025316 | 0.166666667 |
| 5.82 | 0.962025316 | 0.196969697 |
| 5.72 | 0.949367089 | 0.227272727 |
| 5.31 | 0.949367089 | 0.257575758 |
| 5.13 | 0.949367089 | 0.272727273 |
| 4.8 | 0.949367089 | 0.333333333 |
| 4.3 | 0.949367089 | 0.378787879 |
| 4.04 | 0.936708861 | 0.378787879 |
| 3.93 | 0.898734177 | 0.424242424 |
| 3.79 | 0.873417722 | 0.5 |
| 3.33 | 0.835443038 | 0.545454545 |
| 3.29 | 0.835443038 | 0.590909091 |
| 3.03 | 0.835443038 | 0.606060606 |
| 2.79 | 0.835443038 | 0.651515152 |
| 2.73 | 0.810126582 | 0.757575758 |
| 2.54 | 0.797468354 | 0.757575758 |
| 2.43 | 0.797468354 | 0.772727273 |
| 2.29 | 0.708860759 | 0.878787879 |
| 2.12 | 0.556962025 | 0.939393939 |
| 1.82 | 0.506329114 | 0.939393939 |
| 1.79 | 0.341772152 | 0.954545455 |
| 1.52 | 0.240506329 | 1 |
| 1.3 | 0.151898734 | 1 |
| 1.05 | 0.139240506 | 1 |
| 0.91 | 0.050632911 | 1 |
| 0.81 | 0.025316456 | 1 |
| 0 | 0 | 1 |

Fig. 9

Sensitivity and specificity for identifying clusters 1 + 2

| threshold1+2 | sensitivity1+2 | specificity1+2 |
|---|---|---|
| 12.5 | 1 | 0 |
| 11.5 | 1 | 0.075757576 |
| 9.38 | 1 | 0.136363636 |
| 8.3 | 1 | 0.196969697 |
| 8.22 | 1 | 0.318181818 |
| 7.22 | 1 | 0.363636364 |
| 6.99 | 1 | 0.454545455 |
| 6.14 | 0.962025316 | 0.545454545 |
| 5.76 | 0.936708861 | 0.621212121 |
| 5.59 | 0.936708861 | 0.636363636 |
| 5.13 | 0.924050633 | 0.636363636 |
| 5.04 | 0.848101266 | 0.727272727 |
| 4.5 | 0.734177215 | 0.833333333 |
| 4.49 | 0.734177215 | 0.848484848 |
| 3.93 | 0.544303797 | 0.893939394 |
| 3.38 | 0.493670886 | 0.893939394 |
| 3.23 | 0.278481013 | 0.939393939 |
| 2.81 | 0.17721519 | 0.984848485 |
| 1.92 | 0.063291139 | 1 |
| 0.55 | 0.012658228 | 1 |
| 0.48 | 0 | 1 |

Fig. 10

| | AUC |
|---|---|
| CXCL9/MIG (pg/mL) | 0.906214039 |
| CCL4/MIP-1 beta (pg/mL) | 0.893843498 |
| CCL27/CTACK (pg/mL) | 0.879363253 |
| IL-1 beta/IL-1F2 (pg/mL) | 0.864211738 |
| GDF-15 (pg/mL) | 0.852128884 |
| IL-4 (pg/mL) | 0.847909474 |
| G-CSF (pg/mL) | 0.8407173 |
| **IL-6 (pg/mL)** | **0.840141926** |
| CXCL10/IP-10/CRG-2 (pg/mL) | 0.82853855 |
| IL-1 alpha/IL-1F1(pg/mL) | 0.828346759 |
| CCL3/MIP-1 alpha (pg/mL) | 0.788741849 |
| IFN-gamma (pg/mL) | 0.787591101 |
| IL-18/IL-1F4 (pg/mL) | 0.787303414 |
| TNF-alpha (pg/mL) | 0.72765631 |
| IL-1ra/IL-1F3 (pg/mL) | 0.717491369 |
| SCF/c-kit Ligand (pg/mL) | 0.690065209 |
| SCGF/CLEC11a (pg/mL) | 0.67942079 |
| CD25/IL-2R alpha (pg/mL) | 0.640678941 |
| HGF (pg/mL) | 0.605197545 |
| CCL11/Eotaxin(pg/mL) | 0.558879939 |

## INTERNATIONAL SEARCH REPORT

| | |
|---|---|
| International application No. |
| **PCT/JP2022/013767** |

**A. CLASSIFICATION OF SUBJECT MATTER**

*A61K 45/00*(2006.01)i; *A61P 9/12*(2006.01)i; *A61P 11/00*(2006.01)i; *G01N 33/53*(2006.01)i
FI:   G01N33/53 P; A61P11/00; A61P9/12; A61K45/00 ZNA

According to International Patent Classification (IPC) or to both national classification and IPC

**B. FIELDS SEARCHED**

Minimum documentation searched (classification system followed by classification symbols)
   A61K45/00; A61P9/12; A61P11/00; G01N33/53

Documentation searched other than minimum documentation to the extent that such documents are included in the fields searched
   Published examined utility model applications of Japan 1922-1996
   Published unexamined utility model applications of Japan 1971-2022
   Registered utility model specifications of Japan 1996-2022
   Published registered utility model applications of Japan 1994-2022

Electronic data base consulted during the international search (name of data base and, where practicable, search terms used)
   JSTPlus/JMEDPlus/JST7580 (JDreamIII); CAplus/MEDLINE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | 瀧原圭子, 肺動脈性肺高血圧症進展の分子機構, 循環器専門医, 25 March 2012, vol. 20 no. 1, pp. 21-28<br>    section "Cytokines and PAHs", (TAKIHARA, Keiko. Journal of JCS Cardiologists.), non-official translation (Molecular mechanism of development of pulmonary arterial hypertension) | 1-21 |
| A | 中岡良和, Interleukin-6シグナル制御による肺動脈性肺高血圧症の治療の可能性, 循環器専門医, 25 September 2013, vol. 21, no. 2 , pp. 271-276<br>    section "Conclusion", (NAKAOKA, Yoshikazu. Journal of JCS Cardiologists.), non-official translation (Possibility of treatment of pulmonary arterial hypertension by controlling Interleukin-6 signaling) | 1-21 |

☑ Further documents are listed in the continuation of Box C.      ☑ See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **20 May 2022** | **14 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2022/013767**

**C.    DOCUMENTS CONSIDERED TO BE RELEVANT**

| Category* | Citation of document, with indication, where appropriate, of the relevant passages | Relevant to claim No. |
|---|---|---|
| A | 中岡良和, 組織・臓器,個体における動的恒常性とその破綻　I.組織・臓器における動的恒常性とその破綻　3.肺循環の動的恒常性とその破綻　肺動脈性肺高血圧症の発症機構, 実験医学, 01 May 2019, vol. 37, no. 7, pp. 1062-1069<br>　　entire text, all drawings, (NAKAOKA, Yoshikazu. Experimental medicine.), non-official translation (Dynamic homeostasis in tissues, organs and individuals and its collapse I. Dynamic homeostasis in tissues and organs and its collapse 3. Dynamic homeostasis of pulmonary circulation and its collapse mechanism of onset of pulmonary arterial hypertension.) | 1-21 |
| A | JP 2017-519498 A (NOVARTIS AG) 20 July 2017 (2017-07-20)<br>　　claim 1 | 1-21 |
| A | JP 2014-167428 A (JUNTENDO) 11 September 2014 (2014-09-11)<br>　　claim 1 | 1-21 |
| X | QEADAN, Fares et al. The MK2 pathway is linked to G-CSF, cytokine production and metastasis in gastric cancer: a novel intercorrelation analysis approach. Journal of Translational Medicine. 2020, vol. 18, no. 1, 137<br>　　abstract | 14, 18 |
| A | | 1-13, 15-17, 19-21 |
| A | YUAN, Shimin. Interleukin-6 in pulmonary artery hypertension. Jounal of Laboratory Medicine. 2019, vol. 43, no. 4, pp. 177-183<br>　　abstract | 1-21 |

Form PCT/ISA/210 (second sheet) (January 2015)

50

**INTERNATIONAL SEARCH REPORT**

| International application No. |
| --- |
| **PCT/JP2022/013767** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

## INTERNATIONAL SEARCH REPORT

| International application No. |
| --- |
| **PCT/JP2022/013767** |

| Box No. III    Observations where unity of invention is lacking (Continuation of item 3 of first sheet) |
| --- |

This International Searching Authority found multiple inventions in this international application, as follows:

The invention in claim 1 is an invention of a "biomarker for predicting the content of IL-6 in a subject suffering from PAH, whereas claims 14-21 are an invention of a "method for selecting a biomarker", and thus it is clear that there is no common technical feature between these inventions.

Moreover, the close relationship between PAH and IL6, such as the significant increase in IL6 in PAH, the usefulness of IL6 in predicting the prognosis of PAH, and the use of IL6 as a therapeutic target, does not make a contribution over the prior art in light of the disclosures of documents 1 and 2, and thus said technical feature cannot be said to be a special technical feature. Therefore, the claims are classified into three inventions each having the following special technical feature.

(Invention 1) Claims 1, 4, 7, and 10: Invention pertaining to a biomarker for predicting the content of IL-6 in a subject suffering from PAH
(Invention 2) Claims 2-3, 5-6, 8-9, and 11-13: Invention pertaining to a biomarker for predicting the responsiveness of a subject suffering from PAH to a treatment with an IL-6 signaling pathway inhibitory substance
(Invention 3) Claims 14-21: Invention pertaining to a method for selecting a novel biomarker

Document 1: 瀧原圭子, 肺動脈性肺高血圧症進展の分子機構, 循環器専門医, 25 March 2012, vol. 20 no. 1, pp. 21-28, (TAKIHARA, Keiko. Journal of JCS Cardiologists.), non-official translation (Molecular mechanism of development of pulmonary arterial hypertension)
Document 2: 中岡良和, Interleukin-6シグナル制御による肺動脈性肺高血圧症の治療の可能性, 循環器専門医, 25 September 2013, vol. 21, no. 2, pp. 271-276, (NAKAOKA, Yoshikazu. Journal of JCS Cardiologists.), non-official translation (Possibility of treatment of pulmonary arterial hypertension by controlling Interleukin-6 signaling)

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**       ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

Form PCT/ISA/210 (continuation of first sheet) (January 2015)

**INTERNATIONAL SEARCH REPORT**
**Information on patent family members**

International application No.

**PCT/JP2022/013767**

| Patent document cited in search report | | | Publication date (day/month/year) | Patent family member(s) | | | Publication date (day/month/year) |
|---|---|---|---|---|---|---|---|
| JP | 2017-519498 | A | 20 July 2017 | US | 2017/0088897 | A1 | |
| | | | | claim 1 | | | |
| | | | | US | 2020/0002768 | A1 | |
| | | | | WO | 2015/186037 | A1 | |
| | | | | EP | 3152327 | A1 | |
| | | | | CN | 106460059 | A | |
| | | | | KR 10-2017-0013261 | | A | |
| JP | 2014-167428 | A | 11 September 2014 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **YUICHI TAMURA et al.** Ectopic upregulation of membrane-bound IL6R drives vascular remodeling in pulmonary arterial hypertension. *J. Clin. Invest,* May 2018, vol. 128 (5), 1956-1970
- **TAKAHIRO HASHIMOTO-KATAOKA et al.** Interleukin-6/interleukin-21 signaling axis is critical in the pathogenesis of pulmonary arterial hypertension. *PNAS,* 04 May 2015, E2677-E2686
- **TETSUYA TAGA et al.** Interleukin-6 triggers the association of its receptor with a possible signal transducer, gp130. *Cell,* 11 August 1989, vol. 58 (3), 573-581